(19) Europäisches Patentamt
European Patent Office
Office européen des brevets

(11) EP 1 813 284 A1

(12) **EUROPEAN PATENT APPLICATION**
published in accordance with Art. 158(3) EPC

(43) Date of publication:
**01.08.2007 Bulletin 2007/31**

(21) Application number: **05793647.8**

(22) Date of filing: **13.10.2005**

(51) Int Cl.:
*A61K 45/06* (2006.01) *A61K 31/277* (2006.01)
*A61K 31/357* (2006.01) *A61K 31/44* (2006.01)
*A61K 31/4409* (2006.01) *A61K 31/443* (2006.01)
*A61K 31/496* (2006.01) *A61K 31/706* (2006.01)
*A61K 38/00* (2006.01) *A61P 17/00* (2006.01)
*A61P 17/06* (2006.01) *A61K 31/4433* (2006.01)
*A61K 31/4427* (2006.01) *A61K 31/436* (2006.01)
*C07D 405/06* (2006.01) *C07D 405/12* (2006.01)
*C07D 213/30* (2006.01) *C07D 213/75* (2006.01)

(86) International application number:
**PCT/JP2005/018854**

(87) International publication number:
**WO 2006/041120 (20.04.2006 Gazette 2006/16)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.10.2004 JP 2004299104**
**11.04.2005 JP 2005113265**

(71) Applicant: **KYOWA HAKKO KOGYO CO., LTD.**
**Chiyoda-ku,**
**Tokyo 100-8185 (JP)**

(72) Inventors:
- **MANABE, Haruhiko,c/o PHARM. RES.C..,**
**K.H.K.CO.LTD**
**Shizuoka 411-8731 (JP)**
- **OHSHIMA, Etsuo,c/o PHARM. RES.CENT.,**
**K.H.K.CO.LTD**
**Shizuoka 411-8731 (JP)**
- **HARADA, Daisuke,c/o PHARM. RES.CENT.,**
**K.H.K.CO.LTD**
**Shizuoka 411-8731 (JP)**
- **KOBAYASHI, Katsuya,c/o PHARM. RES.C.,**
**K.H.K.CO.LTD**
**Shizuoka 411-8731 (JP)**

(74) Representative: **Casalonga, Axel**
**Bureau Casalonga & Josse**
**Bayerstrasse 71/73**
**80335 München (DE)**

(54) **PHARMACEUTICAL COMPOSITION**

(57) The present invention provides a pharmaceutical composition comprising (a) a phosphodiesterase (PDE)-IV inhibitor or a pharmaceutically acceptable salt thereof and (b) an immunosuppressant, a therapeutic and/or preventive agent for chronic skin diseases comprising (a) a PDE-IV inhibitor or a pharmaceutically acceptable salt thereof and (b) an immunosuppressant, a therapeutic and/or preventive agent for chronic skin diseases to be administered simultaneously or separately with an interval comprising (a) a PDE-IV inhibitor or a pharmaceutically acceptable salt thereof and (b) an immunosuppressant, as active ingredients,; and the like.



Printed by Jouve, 75001 PARIS (FR)

## Description

Technical Field

The present invention relates to a pharmaceutical composition and the like comprising a phosphodiesterase (PDE)-IV inhibitor and an immunosuppressant.

Background Art

Phosphodiesterase (PDE) degrades cyclic adenosine 3',5'-monophosphate (cAMP) or cyclic guanosine 3',5'-monophosphate (cGMP) to regulate their concentrations in cells. PDE-IV, which is one of the PDE isozymes, is expressed in keratinocytes and inflammatory cells such as monocytes, macrophages, B-cells, T-cells and eosinophils (Br. J. Pharmacol., 1997, 121, p.221; J. Invest. Dermatol., 1985, 84, p.477; J. Pharmacol. Exp. Ther., 1994, 271, p.1167; J. Invest. Dermatol., 1998, 110, p287), and regulates cAMP or cGMP concentration. PDE-IV plays an important role for controlling inflammatory responses; i.e. regulation of the infiltration of inflammatory cells into inflammatory sites, the activation of the inflammatory cells, the activation of keratinocytes and the like (Mol. Pharmacol., 1995, 47, p.1164; Clin. Exp. Allergy, 1995, 25, p.616).

On the other hand, chronic skin diseases are considered to be induced or worsened by infiltration of inflammatory cells into skin lesions, activation of inflammatory cells in skin lesions, or activation of keratinocytes (J. Allergy Clin. Immunol., 2001, 107, p.871). Therefore, PDE-IV inhibitors are expected as therapeutic and/or preventive agents for chronic skin diseases.

For example, SB207499, which is one of the PDE-IV inhibitors, has been reported to inhibit a delayed-type allergic reaction in the skin in an animal model (refer to Non-patent Document 1). SB207499 is also reported to exhibit therapeutic effects on chronic dermatitis models (refer to Non-patent Document 2).

Hitherto, compounds represented by Formulae (I) to (XIV) or pharmaceutically acceptable salts thereof have been known to be used as PDE-IV inhibitors (refer to Patent Documents 1 to 9, Non-Patent Documents 1 to 8).

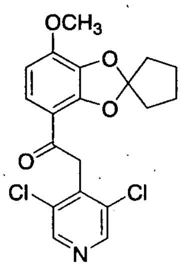
( I )

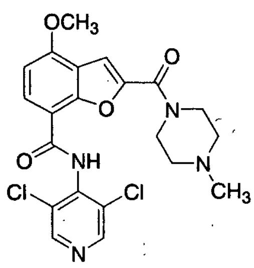
( II )

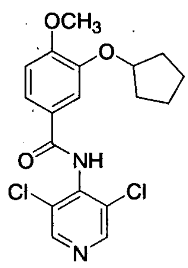
( III )

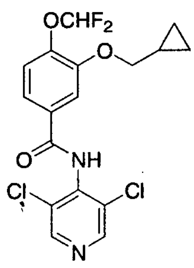
( IV )

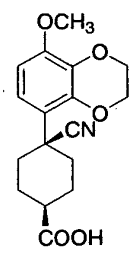
( V )

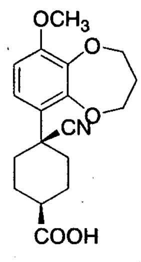
( VI )

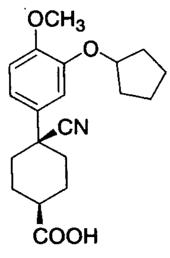
( VII )

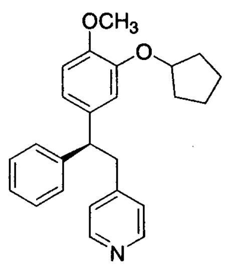
( VIII )

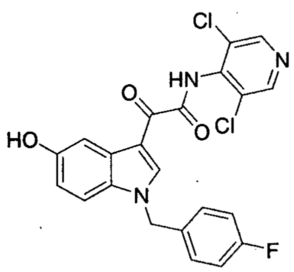
( IX )

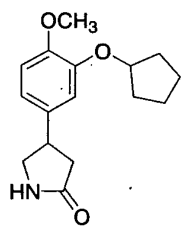
( X )

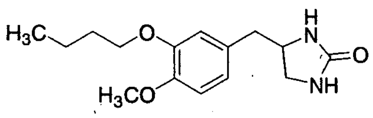

( XI )

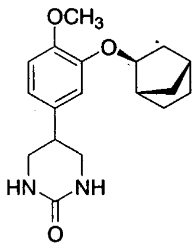

( XII )

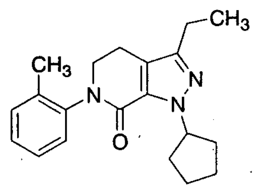

( XIII )

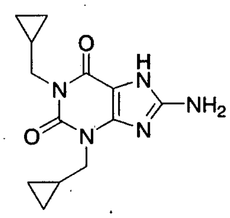

( XIV )

On the other hand, unlike mechanisms of steroid agents and the like, immunosuppressant has been widely known to show suppressive activity on inflammatory cells such as T-cell, mast cell, and eosinophil in vitro, and suppressive activity on dermatitis model in vivo [Folia Pharmacol. Jpn., 2001, vol.117, p.351-357]. Therefore, immunosuppressant has gotton a lot of attention recently as a therapeutic and/or preventive agent for chronic skin diseases [Lancet, 2003, vol.361, p.151-160; Expert Opin. Emerging Drugs, 2002, vol.7, p.69-90].

Patent Document 1: WO 96/36624
Patent Document 2: WO 99/16768
Patent Document 3: WO 95/01338
Patent Document 4: WO 00/14085
Patent Document 5: WO 94/14742
Patent Document 6: WO 99/55696
Patent Document 7: WO 92/19594
Patent Document 8: US Patent No. 3636039
Patent Document 9: WO 87/06576
Non-Patent Document 1: Eur. J. Pharmacol., 2002, vol.446, p.195
Non-Patent Document 2: J. Pharmacol. Exp. Ther., 1998, vol.287, p.705
Non-Patent Document 3: J. Med.Chem., 1994, vol.37, p.1696
Non-Patent Document 4: J. Med.Chem., 1998, vol.41, p.821
Non-Patent Document 5: J. Med.Chem., 1998, vol.41, p.2268
Non-Patent Document 6: Br. J. Dermatol., 2002, vol.147, p.299)
Non-Patent Document 7: J. Am. Acad. Dermatol., 1999, vol.41, p.72)
Non-Patent Document 8: Exp. Opin. Invest. Drugs, 1999, vol.8, p.1301-1325)

Disclosure of the Invention

Problems to be Solved by the Invention

An object of the present invention is to provide a pharmaceutical composition and the like useful as a therapeutic and/or preventive agent for chronic skin diseases (for example, contact dermatitis, atopic dermatitis, seborrheic dermatitis, nummular eczema, lichen simplex chronicus Vidal, autosensitization dermatitis, stasis dermatitis, asteatotic eczema, and psoriasis) .

Means of Solving the Problems

The present invention relates to the following (1) to (64).

(1) A pharmaceutical composition which comprises (a) a PDE-IV inhibitor or a pharmaceutically acceptable salt thereof and (b) an immunosuppressant.
(2) The pharmaceutical composition according to (1), wherein the PDE-IV inhibitor is a compound selected from a group consisting of compounds represented by Formulae (I) to (XIV):

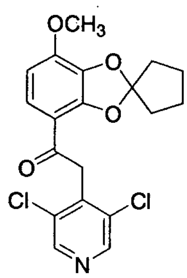
( I )

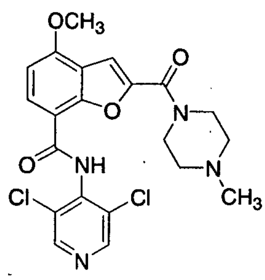
( II )

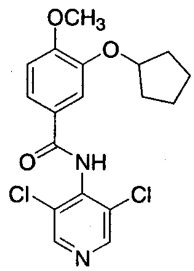
( III )

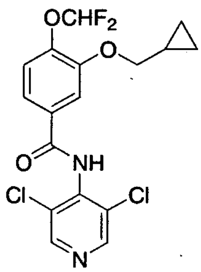
( IV )

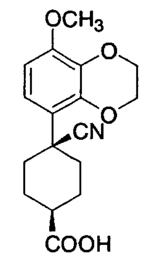
( V )

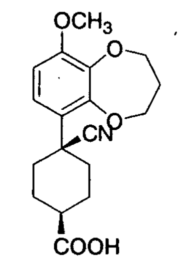
( VI )

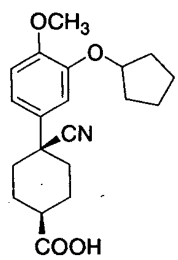
( VII )

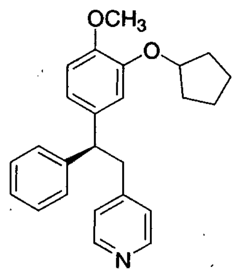
( VIII )

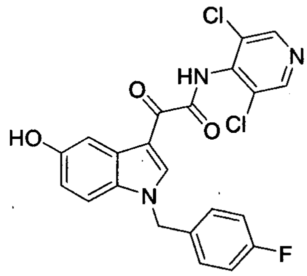
( IX )

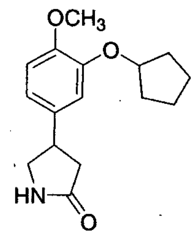
( X )

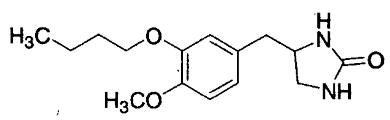
( XI )

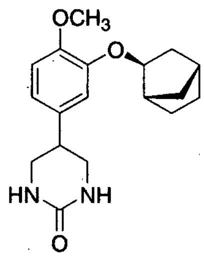
( XII )

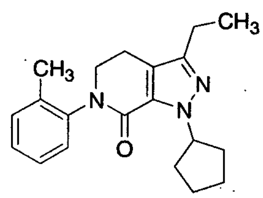
( XIII )

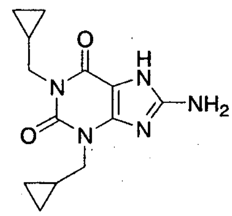
( XIV )

(3) The pharmaceutical composition according to (1), wherein the PDE-IV inhibitor is a compound represented by Formula (I):

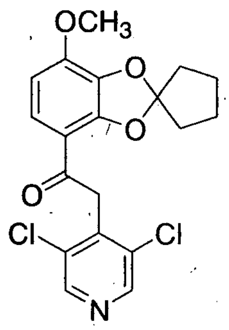
( I )

(4) The pharmaceutical composition according to any one of (1) to (3), wherein the immunosuppressant is an immunosuppressant selected from a group consisting of Tacrolimus, Pimecrolimus, Ascomycin, Rapamycin, FTY

720, Azathioprine, Cyclophosphamide, Methotrexate, Mizoribine and Cyclosporin A.

(5) A therapeutic and/or preventive agent for chronic skin diseases which comprises (a) a PDE-IV inhibitor or a pharmaceutically acceptable salt thereof and (b) an immunosuppressant, as active ingredients.

(6) The therapeutic and/or preventive agent for chronic skin diseases according to (5), wherein the PDE-IV inhibitor is a compound selected from a group consisting of compounds represented by Formulae (I) to (XIV):

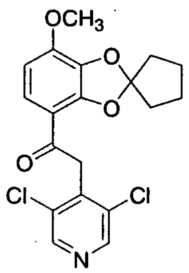
( I )

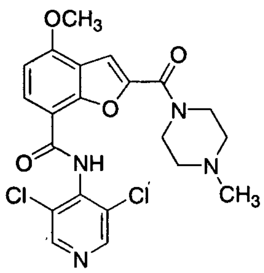
( II )

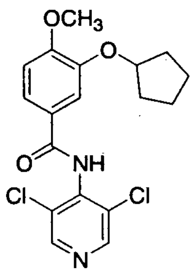
( III )

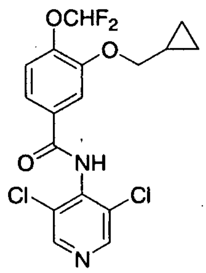
( IV )

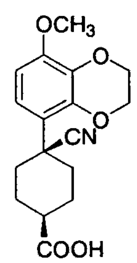
( V )

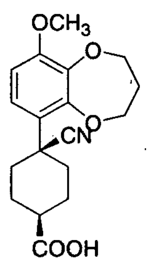
( VI )

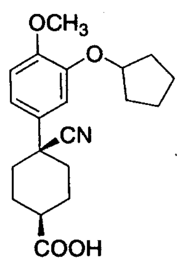
( VII )

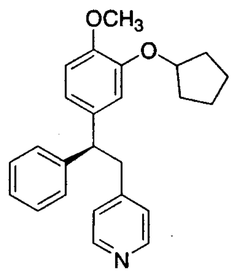
( VIII )

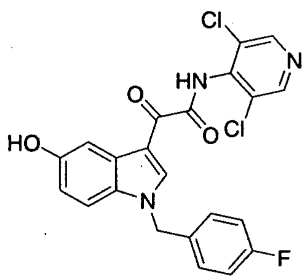
( IX )

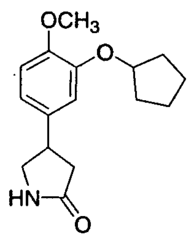
( X )

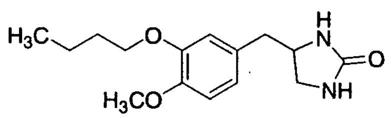
( XI )

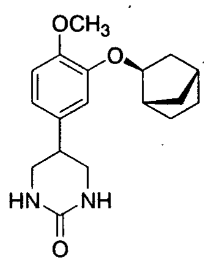
( XII )

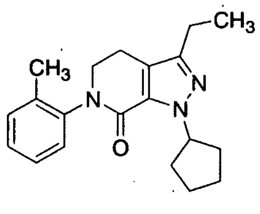
( XIII )

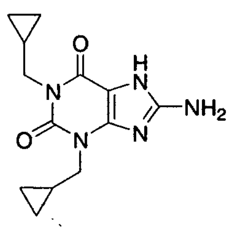
( XIV )

(7) The therapeutic and/or preventive agent for chronic skin diseases according to (5), wherein the PDE-IV inhibitor is a compound represented by Formula (I):

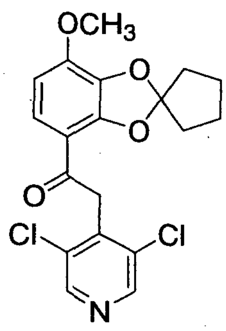

( I )

(8) The therapeutic and/or preventive agent for chronic skin diseases according to any one of (5) to (7), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

(9) The therapeutic and/or preventive agent for chronic skin diseases according to any one of (5) to (8), wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

(10) The therapeutic and/or preventive agent for chronic skin diseases according to any one of (5) to (9), wherein the agent is an external preparation.

(11) A therapeutic and/or preventive agent for chronic skin diseases for administering simultaneously or separately with an interval, which comprises (a) a PDE-IV inhibitor or a pharmaceutically acceptable salt thereof and (b) an immunosuppressant, as active ingredients.

(12) The therapeutic and/or preventive agent for chronic skin diseases according to (11), wherein the PDE-IV inhibitor is a compound selected from a group consisting of compounds represented by Formulae (I) to (XIV):

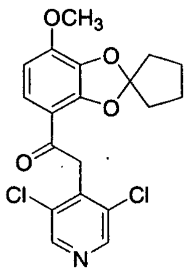

( I )

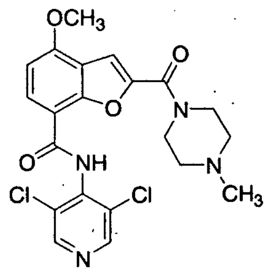

( II )

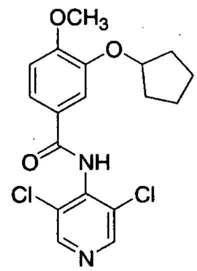

( III )

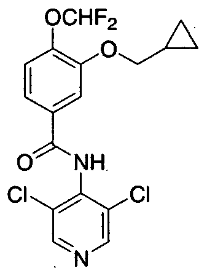

( IV )

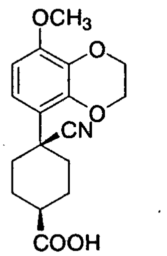

( V )

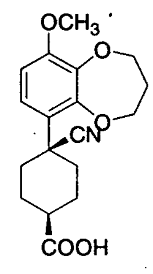

( VI )

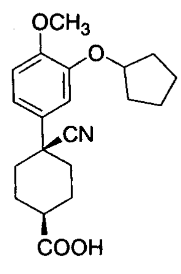

( VII )

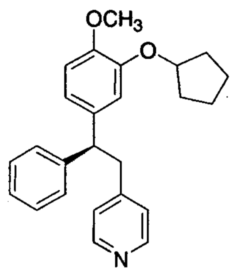

( VIII )

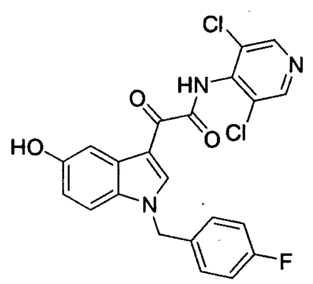

( IX )

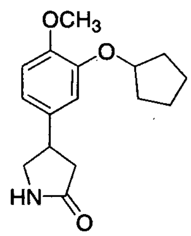

( X )

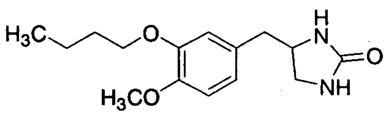
( XI )

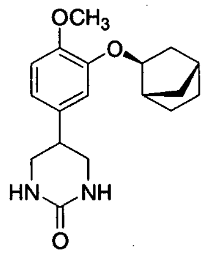
( XII )

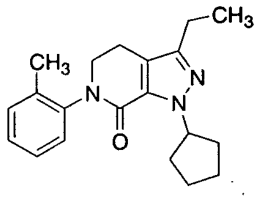
( XIII )

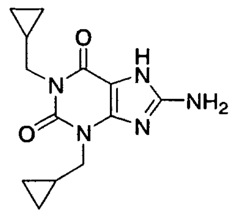
( XIV )

(13) The therapeutic and/or preventive agent for chronic skin diseases according to (11), wherein the PDE-IV inhibitor is a compound represented by Formula (I):

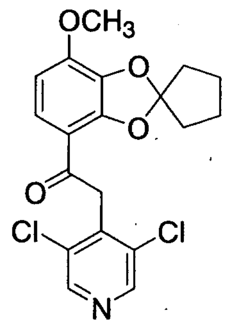
( I )

(14) The therapeutic and/or preventive agent for chronic skin diseases according to any one of (11) to (13), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.
(15) The therapeutic and/or preventive agent for chronic skin diseases according to any one of (11) to (14), wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.
(16) The therapeutic and/or preventive agent for chronic skin diseases according to any one of (11) to (15), wherein the agent is an external preparation.
(17) A kit characterized by comprising (a) a first component comprising a PDE-IV inhibitor or a pharmaceutically acceptable salt thereof and (b) a second component comprising an immunosuppressant.
(18) The kit according to (17), wherein the PDE-IV inhibitor is a compound selected from a group consisting of compounds represented by Formulae (I) to (XIV):

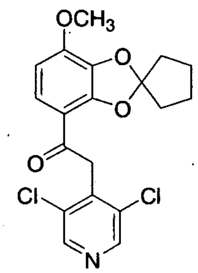
( I )

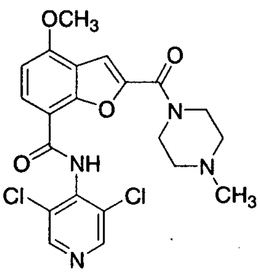
( II )

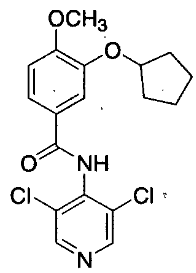
( III )

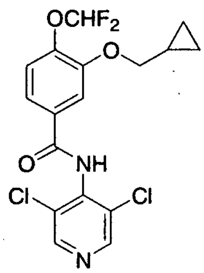
( IV )

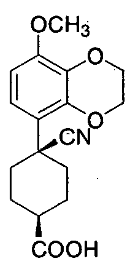
( V )

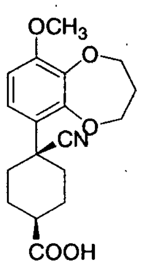
( VI )

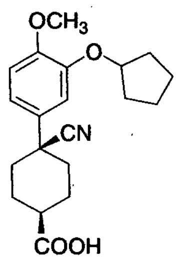
( VII )

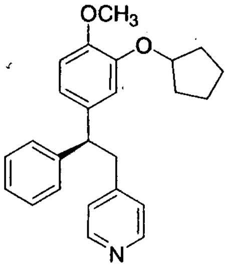
( VIII )

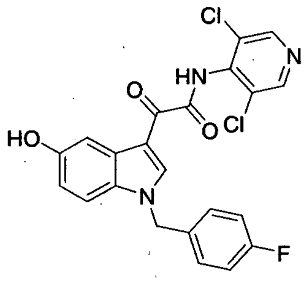
( IX )

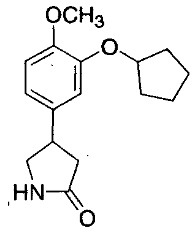
( X )

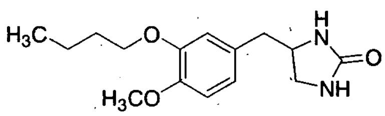
( XI )

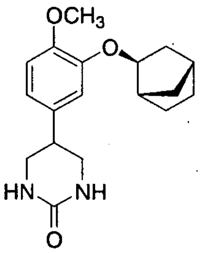
( XII )

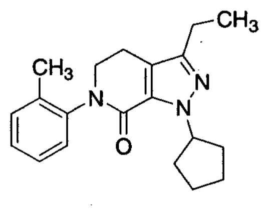
( XIII )

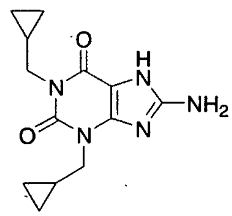
( XIV )

(19) The kit according to (17), wherein the PDE-IV inhibitor is a compound represented by Formula (I):

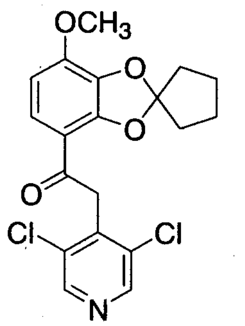
( I )

(20) The kit according to any one of (17) to (19), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.
(21) A kit for treating and/or preventing chronic skin diseases characterized by comprising (a) a first component comprising a PDE-IV inhibitor or a pharmaceutically acceptable salt thereof and (b) a second component comprising an immunosuppressant.
(22) The kit for treating and/or preventing chronic skin diseases according to (21), wherein the PDE-IV inhibitor is a compound selected from a group consisting of compounds represented by Formulae (I) to (XIV):

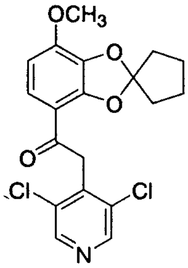
( I )

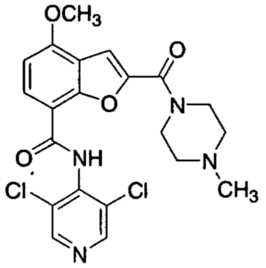
( II )

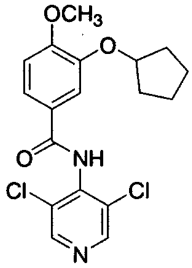
( III )

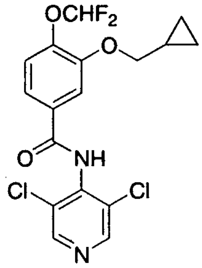
( IV )

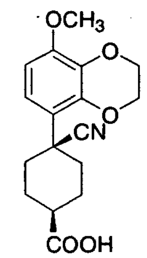
( V )

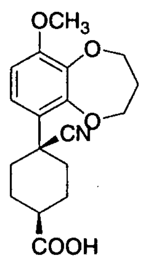
( VI )

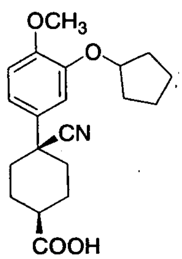
( VII )

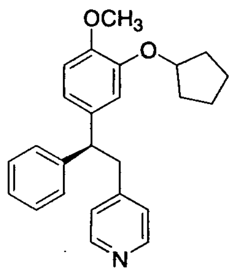
( VIII )

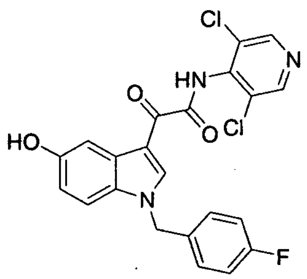
( IX )

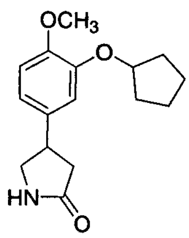
( X )

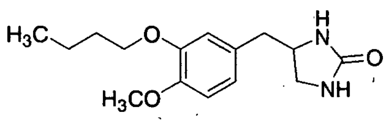
( XI )

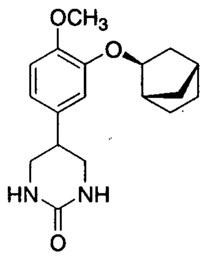
( XII )

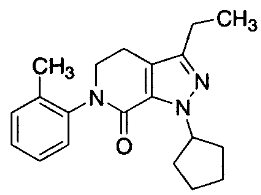
( XIII )

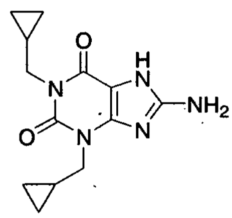
( XIV )

(23) The kit for treating and/or preventing chronic skin diseases according to (21), wherein the PDE-IV inhibitor is a compound represented by Formula (I):

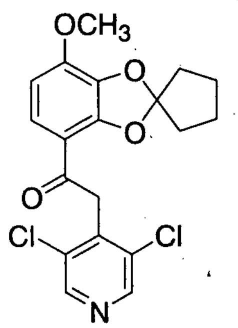
( I )

(24) The kit for treating and/or preventing chronic skin diseases according to any one of (21) to (23), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.
(25) The kit for treating and/or preventing chronic skin diseases according to any one of (21) to (24), wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.
(26) The kit for treating and/or preventing chronic skin diseases according to any one of (21) to (25), wherein the kit is a kit of external preparations.
(27) A PDE-IV inhibitor or a pharmaceutically acceptable salt thereof for administering simultaneously or separately with an interval with an immunosuppressant.
(28) The PDE-IV inhibitor or a pharmaceutically acceptable salt thereof according to (27), wherein the PDE-IV inhibitor is a compound selected from a group consisting of compounds represented by Formulae (I) to (XIV):

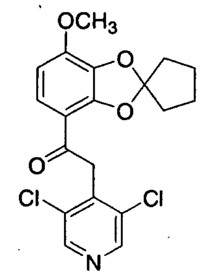
( I )

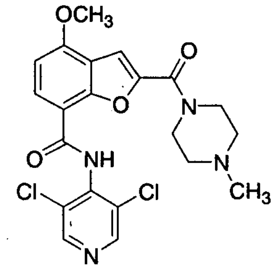
( II )

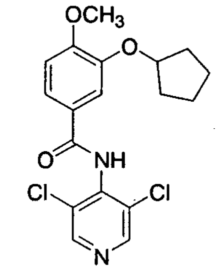
( III )

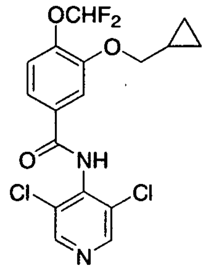
( IV )

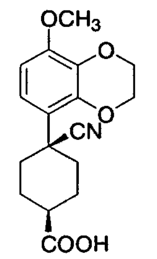
( V )

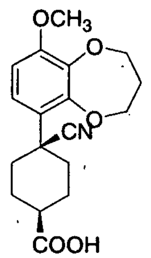
( VI )

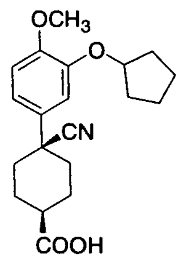
( VII )

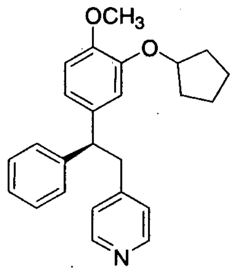
( VIII )

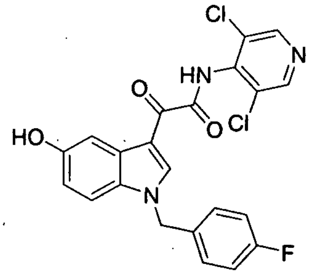
( IX )

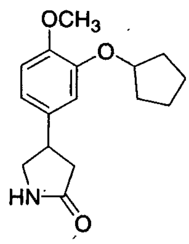
( X )

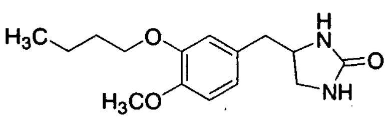
( XI )

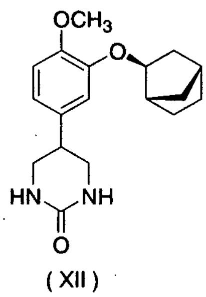
( XII )

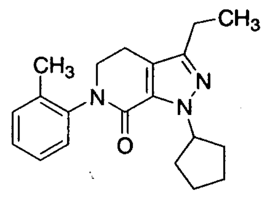
( XIII )

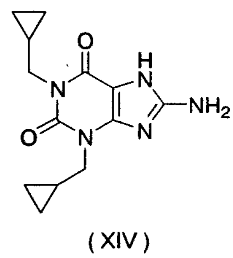
( XIV )

(29) The PDE-IV inhibitor or a pharmaceutically acceptable salt thereof according to (27), wherein the PDE-IV inhibitor is a compound represented by Formula (I):

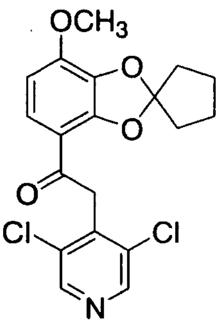

( I )

(30) The PDE-IV inhibitor or a pharmaceutically acceptable salt thereof according to any one of (27) to (29), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

(31) A pharmaceutical composition which comprises, as an active ingredient, a PDE-IV inhibitor or a pharmaceutically acceptable salt thereof for administering simultaneously or separately with an interval with an immunosuppressant.

(32) The pharmaceutical composition according to (31), wherein the PDE-IV inhibitor is a compound selected from a group consisting of compounds represented by Formulae (I) to (XIV):

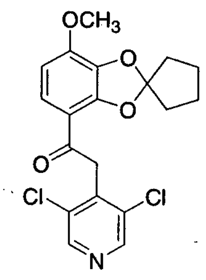

( I )

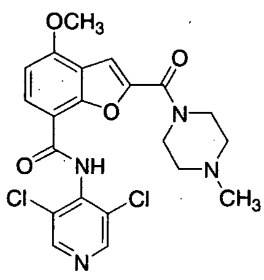

( II )

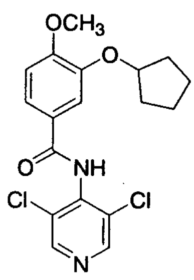

( III )

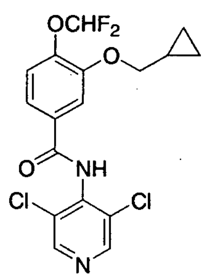

( IV )

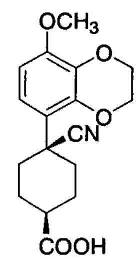

( V )

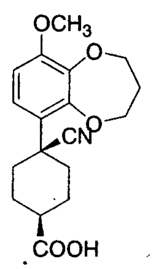

( VI )

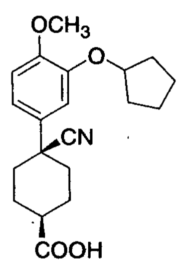

( VII )

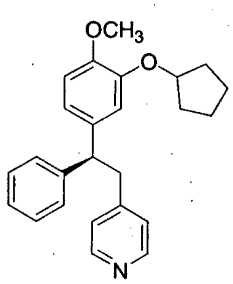

( VIII )

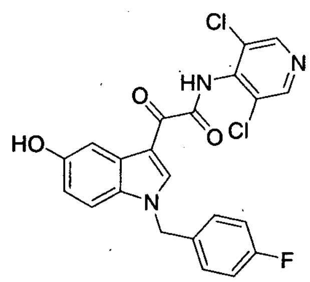

( IX )

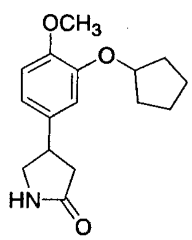

( X )

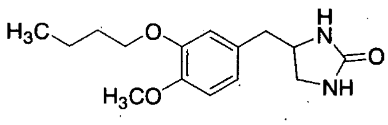
( XI )

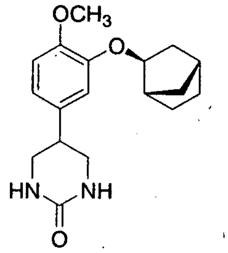
( XII )

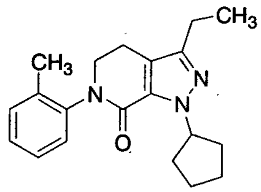
( XIII )

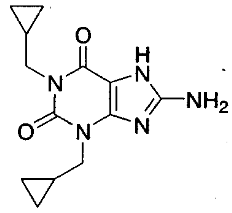
( XIV )

(33) The pharmaceutical composition according to (31), wherein the PDE-IV inhibitor is a compound represented by Formula (I):

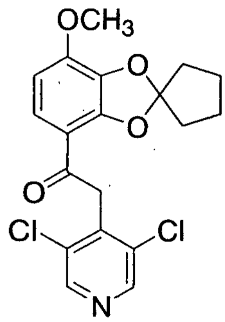
( I )

(34) The pharmaceutical composition according to any one of (31) to (33), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

(35) A method for treating and/or preventing chronic skin diseases characterized by administering (a) a PDE-IV inhibitor or a pharmaceutically acceptable salt thereof and (b) an immunosuppressant simultaneously or separately with an interval.

(36) The method for treating and/or preventing chronic skin diseases according to (35), wherein the PDE-IV inhibitor is a compound selected from a group consisting of compounds represented by Formulae (I) to (XIV):

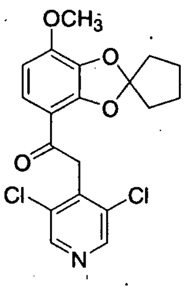
( I )

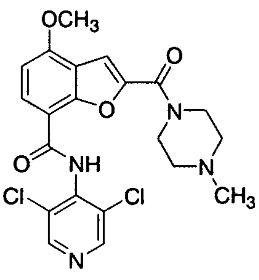
( II )

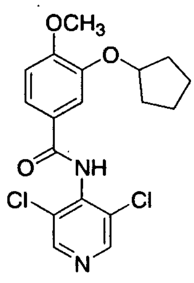
( III )

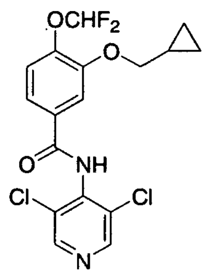
( IV )

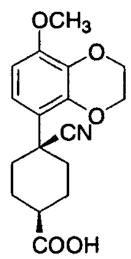
( V )

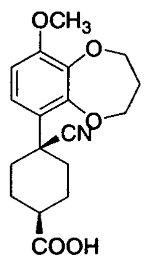
( VI )

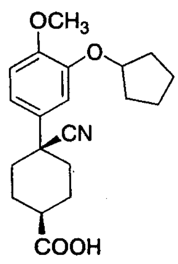
( VII )

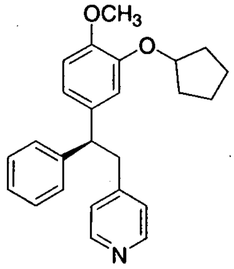
( VIII )

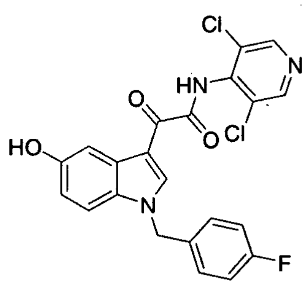
( IX )

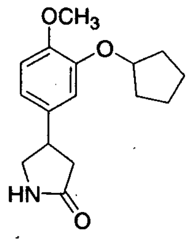
( X )

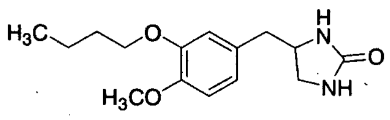
( XI )

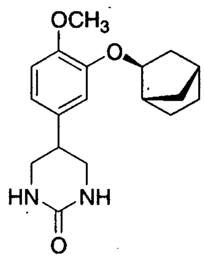
( XII )

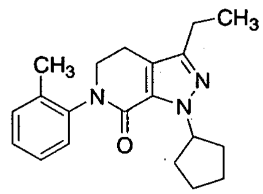
( XIII )

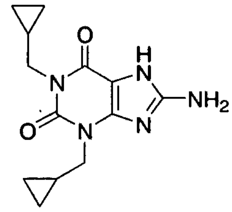
( XIV )

(37) The method for treating and/or preventing chronic skin diseases according to (35), wherein the PDE-IV inhibitor is a compound represented by Formula (I):

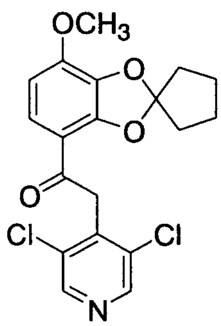
( I )

(38) The method for treating and/or preventing chronic skin diseases according to any one of (35) to (37), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.
(39) The method for treating and/or preventing chronic skin diseases according to any one of (35) to (38), wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.
(40) The method for treating and/or preventing chronic skin diseases according to any one of (35) to (39), characterized by administering (a) a PDE-IV inhibitor or a pharmaceutically acceptable salt thereof and (b) an immunosuppressant as external preparations.
(41) A method for treating and/or preventing chronic skin diseases characterized by administering an effective amount of a combination of (a) a PDE-IV inhibitor or a pharmaceutically acceptable salt thereof and (b) an immunosuppressant.
(42) The method for treating and/or preventing chronic skin diseases according to (41), wherein the PDE-IV inhibitor is a compound selected from a group consisting of compounds represented by Formulae (I) to (XIV):

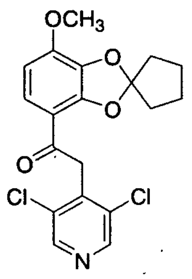

( I )

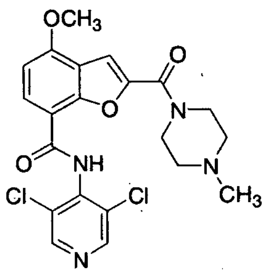

( II )

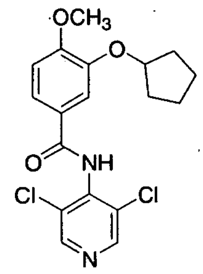

( III )

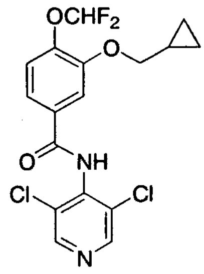

( IV )

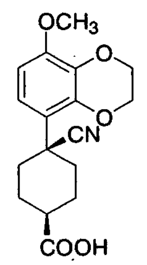

( V )

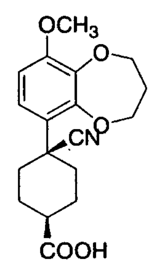

( VI )

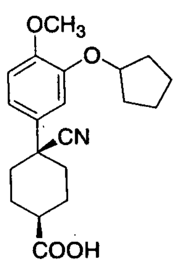

( VII )

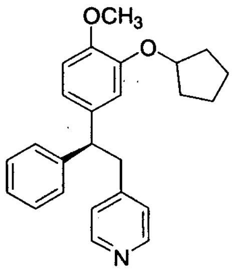

( VIII )

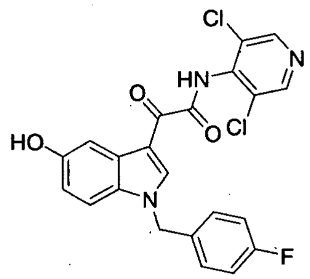

( IX )

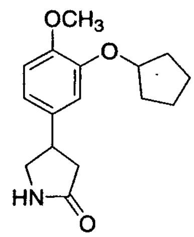

( X )

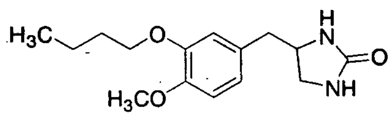

( XI )

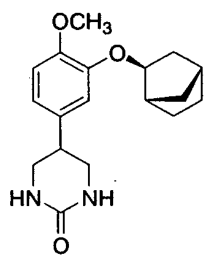

( XII )

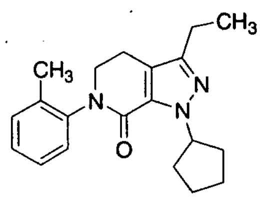

( XIII )

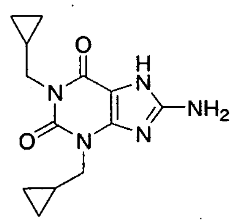

( XIV )

(43) The method for treating and/or preventing chronic skin diseases according to (41), wherein the PDE-IV inhibitor is a compound represented by Formula (I):

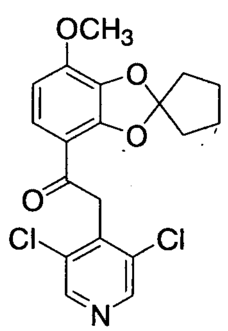

( I )

(44) The method for treating and/or preventing chronic skin diseases according to any one of (41) to (43), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

(45) The method for treating and/or preventing chronic skin diseases according to any one of (41) to (44), wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

(46) The method for treating and/or preventing chronic skin diseases according to any one of (41) to (45), wherein the administrating is an administration of external preparations.

(47) Use of (a) a PDE-IV inhibitor or a pharmaceutically acceptable salt thereof and (b) an immunosuppressant for the manufacture of a therapeutic and/or preventive agent for chronic skin diseases.

(48) Use according to (47), wherein the PDE-IV inhibitor is a compound selected from a group consisting of compounds represented by Formulae (I) to (XIV):

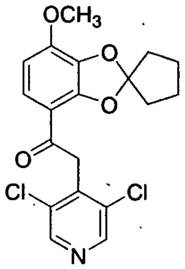
( I )

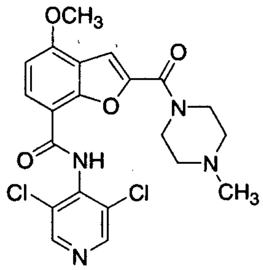
( II )

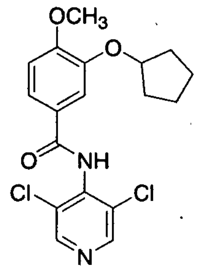
( III )

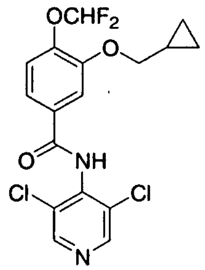
( IV )

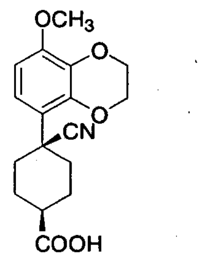
( V )

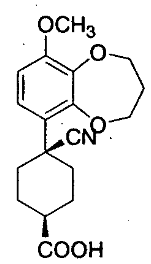
( VI )

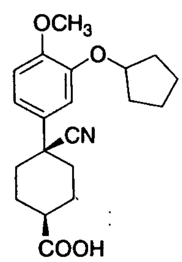
( VII )

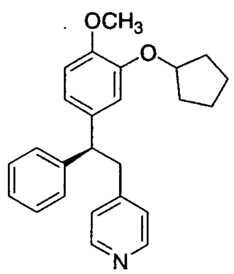
( VIII )

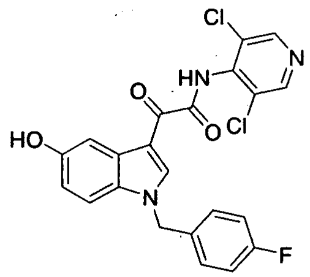
( IX )

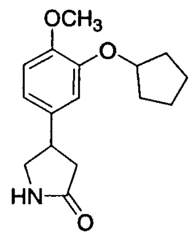
( X )

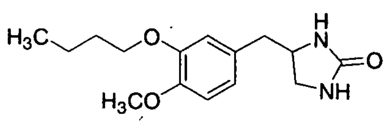
( XI )

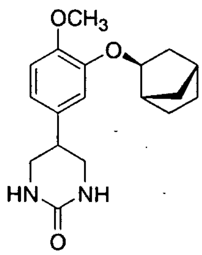
( XII )

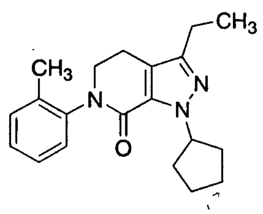
( XIII )

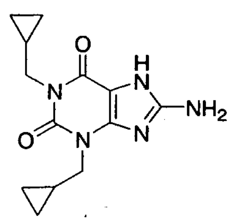
( XIV )

(49) Use according to (47), wherein the PDE-IV inhibitor is a compound represented by Formula (I):

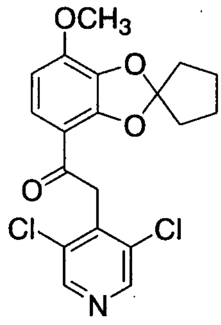

( I )

(50) Use according to any one of (47) to (49), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

(51) Use according to any one of (47) to (50), wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

(52) Use according to any one of (47) to (51), wherein the therapeutic and/or preventive agent for chronic skin diseases is an external preparation.

(53) Use of (a) a PDE-IV inhibitor or a pharmaceutically acceptable salt thereof and (b) an immunosuppressant for the manufacture of a therapeutic and/or preventive agent for chronic skin diseases to be administered simultaneously or separately with an interval, which comprises (a) and (b), as active ingredients.

(54) Use according to (53), wherein the PDE-IV inhibitor is a compound selected from a group consisting of compounds represented by Formulae (I) to (XIV):

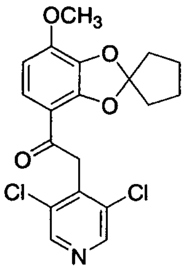

( I )

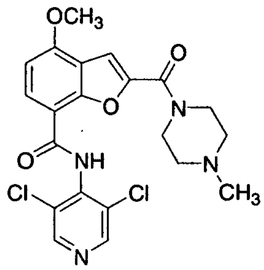

( II )

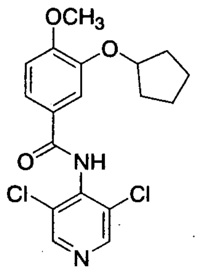

( III )

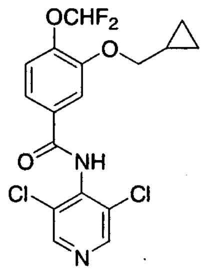

( IV )

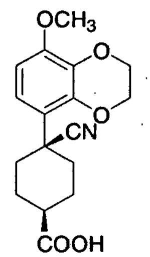

( V )

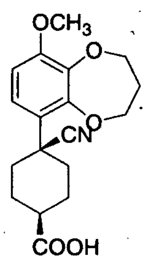

( VI )

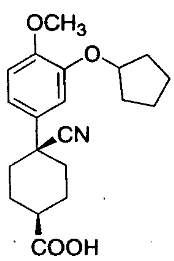

( VII )

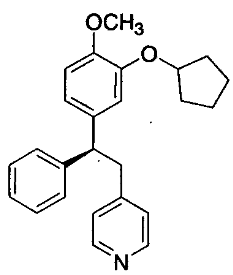

( VIII )

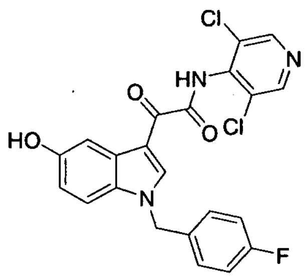

( IX )

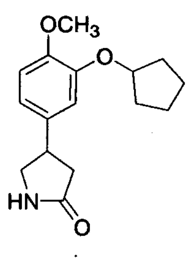

( X )

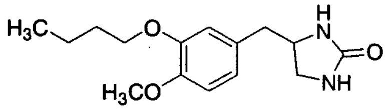
( XI )

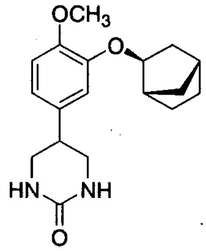
( XII )

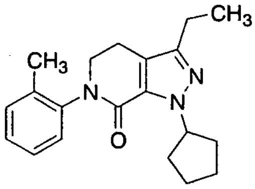
( XIII )

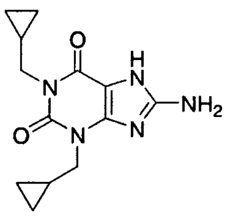
( XIV )

(55) Use according to (53), wherein the PDE-IV inhibitor is a compound represented by Formula (I):

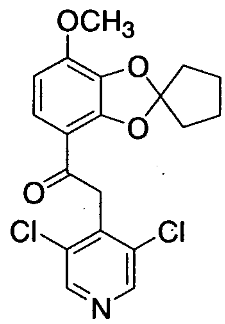
( I )

(56) Use according to any one of (53) to (55), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.
(57) Use according to any one of (53) to (56), wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.
(58) Use according to any one of (53) to (57), wherein the therapeutic and/or preventive agent for chronic skin diseases is an external preparation.
(59) Use of (a) a PDE-IV inhibitor or a pharmaceutically acceptable salt thereof and (b) an immunosuppressant, for the manufacture of a kit for treating and/or preventing chronic skin diseases which comprises a first component comprising (a) and a second component comprising (b).
(60) Use according to (59), wherein the PDE-IV inhibitor is a compound selected from a group consisting of compounds represented by Formulae (I) to (XIV):

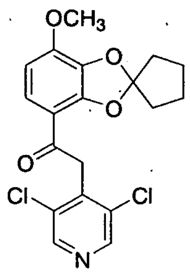
( I )

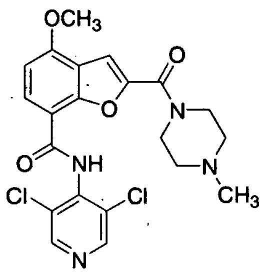
( II )

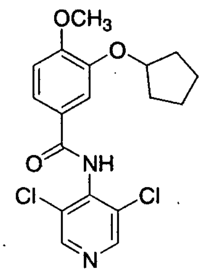
( III )

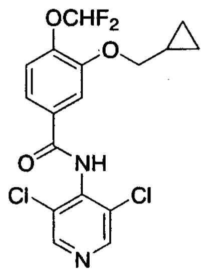
( IV )

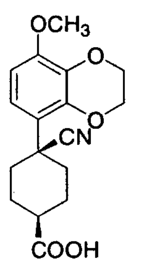
( V )

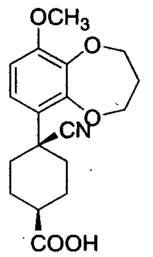
( VI )

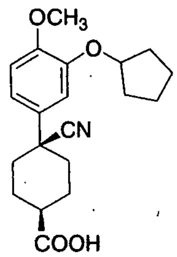
( VII )

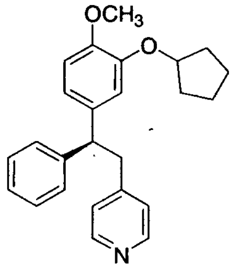
( VIII )

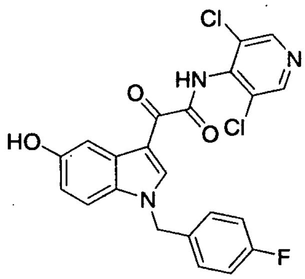
( IX )

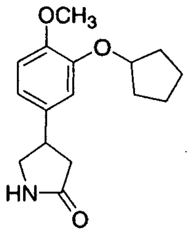
( X )

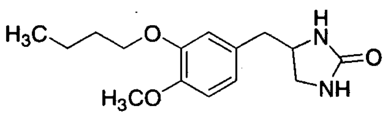
( XI )

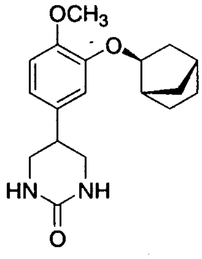
( XII )

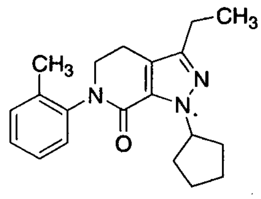
( XIII )

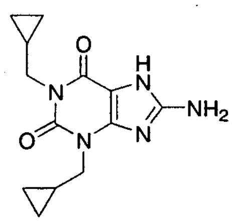
( XIV )

(61) Use according to (59), wherein the PDE-IV inhibitor is a compound represented by Formula (I):

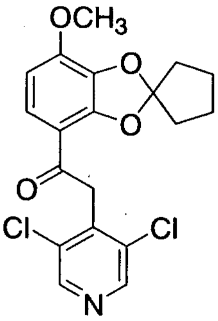
( I )

(62) Use according to any one of (59) to (61), wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.
(63) Use according to any one of (59) to (62), wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.
(64) Use according to any one of (59) to (63), wherein the kit for treating and/or preventing chronic skin diseases is a kit of external preparations.

Effect of the Invention

The present invention provides a pharmaceutical composition and the like comprising (a) a PDE-IV inhibitor or a pharmaceutically acceptable salt thereof and (b) an immunosuppressant as active ingredients.

Best Mode for Carrying out the Invention

The PDE-IV inhibitor to be used for the present invention and the PDE-IV inhibitor of the present invention are not particularly limited so long as they are compounds having PDE-IV inhibitory activity. Among them, a compound having selective PDE-IV inhibitory activity is preferred, a compound in which $IC_{50}$ value of the inhibitory activity is 1 μmol/L or less is more preferred, and a compound in which $IC_{50}$ value of the inhibitory activity is 0.1 μmol/L or less is further preferred. In addition, when administered orally or parenterally, a compound having no side effects such as vomiting is preferred. And a compound having suitable physical property to be used as external preparation is further preferred. Specifically, the compounds include those represented by the following Formulae (I) to (XIV), such as 7-[2-(3,5-dichloro-4-pyridyl)-1-oxoethyl]-4-methoxy-spiro[1,3-benzodioxol-2,1'-cyclopentane] (the following Formula (I)), 4-[(3,5-dichloro-4-pyridyl)carbamoyl]-7-methoxy-2-(4-methylpiperazine-1-ylcarbonyl)benzofuran (the following Formula(II)), piclamilast (the following Formula (III)), roflumilast (the following Formula (IV)), cis-4-cyano-4-(8-methoxy-1,4-benzodioxan-5-yl)cyclohexane carboxylic acid (the following Formula (V)), cis-4-cyano-4-(3,4-dihydro-9-methoxy-2H-1,5-benzodioxepin-6-yl)cyclohexane carboxylic acid (the following Formula (VI)), ariflo (the following Formula (VII)), CDP840 (the following Formula (VIII)), AWD12-281 (the following Formula (IX)), rolipram (the following Formula (X)), Ro20-1724 (the following Formula (XI)), atizoram (the following Formula (XII)), CP220629 (the following Formula (XIII)), cipamfylline (the following Formula (XIV)), GK-07294, atopik and IPL-4088. Among them, 7-[2-(,3,5-dichloro-4-pyridyl)-1-oxoethyl]-4-methoxy-spiro[1,3-benzodioxol-2,1'-cyclopentane] (Formula (I)) is preferred.

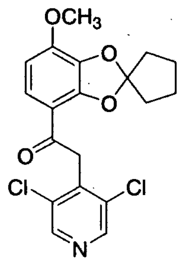
(I)

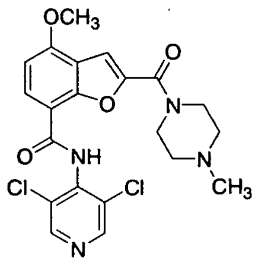
(II)

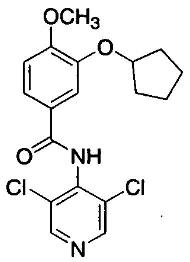
(III)

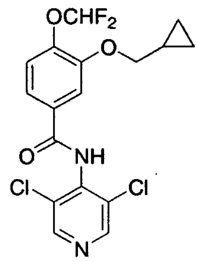
(IV)

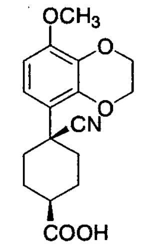
(V)

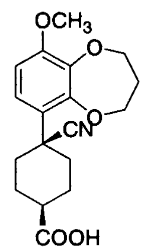
(VI)

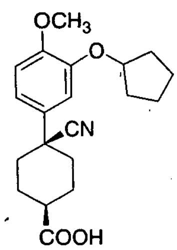
(VII)

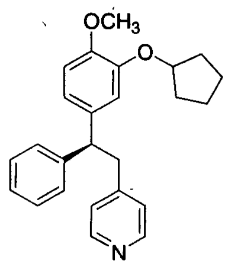
(VIII)

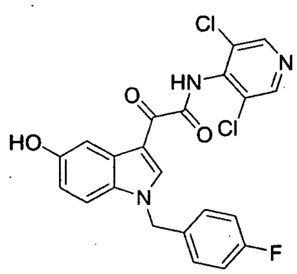
(IX)

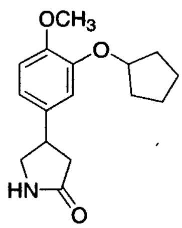
(X)

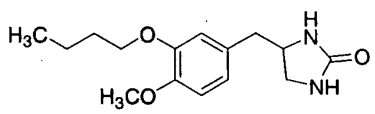
(XI)

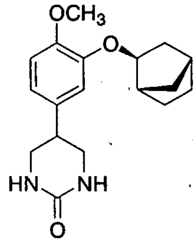
(XII)

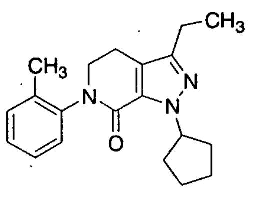
(XIII)

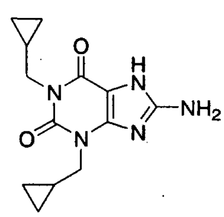
(XIV)

Herein after, the compound represented by Formula (I) is referred to as Compound (I). The compounds having the other formula numbers are referred to in the same manner.

Examples of the pharmaceutically acceptable salt of the PDE-IV inhibitor used in the present invention and the

PDE-IV inhibitor of the present invention include pharmaceutically acceptable acid addition salts, metal salts, ammonium salts, organic amine addition salts, amino acid addition salts and the like.

Examples of the pharmaceutically acceptable acid addition salts of the PDE-IV inhibitor used in the present invention and the PDE-IV inhibitor of the present invention include inorganic acid salts such as a hydrochloride, a sulfate, a hydrobromate, a nitrate, a phosphate and the like; and organic acid salts such as an acetate, a mesylate, a succinate, a maleate, a fumarate, a citrate, a tartrate, and the like. Examples of the pharmaceutically acceptable metal salts include alkali metal salts such as a sodium salt, a potassium salt and the like; alkaline-earth metal salts such as a magnesium salt, a calcium salt and the like; an aluminum salt; a zinc salt and the like. Examples of the pharmaceutically acceptable ammonium salts include salts of ammonium, tetramethylammonium or the like. Examples of the pharmaceutically acceptable organic amine addition salts include addition salts of morpholine, piperidine or the like. Examples of the pharmaceutically acceptable amino acid addition salts include addition salts of glycine, phenylalanine, lysine, aspartic acid, glutamic acid or the like.

The PDE-IV inhibitor used for the present invention and the PDE-IV inhibitor of the present invention can be prepared according to known methods, respectively. For example, Compound (I) can be prepared by the method disclosed in WO 96/36624 and the like. Compound (II) can be prepared by the method disclosed in WO 96/36624, WO99/16768 and the like. Compound (III) can be prepared by the method disclosed in J. Med. Chem., 1994, vol.37, p.1696 and the like. Compound (IV) can be prepared by the method disclosed in WO 95/01338 and the like. Compounds (V) and (VI) can be prepared by the method disclosed in WO 98/22455, WO00/14085 and the like. Compound (VII) can be prepared by the method disclosed in J. Med. Chem., 1998, vol.41, p.821 and the like. Compound (VIII) can be prepared by the method disclosed in WO 94/14742, WO95/17386 and the like. Compound (IX) can be prepared by the method disclosed in WO 99/55696 and the like. Compound (X) can be prepared by the method disclosed in WO92/19594 and the like. Compound (XI) can be prepared by the method disclosed in US 3636039 and the like. Compound (XII) can be prepared by the method disclosed in WO87/05676 and the like. Compound (XIII) can be prepared by the method disclosed in J. Med. Chem., 1998, vol.41, p.2268 and the like. Compound (XIV) can be prepared by the method disclosed in EP 389282 and the like.

Among the PDE-IV inhibitors used in the present invention and the PDE-IV inhibitors of the present invention, tautomers, stereoisomers, and the like may be existed. However, all possible isomers including these and the mixtures thereof can be used for the pharmaceutical composition, the therapeutic and/or preventive agent for chronic skin diseases, the kit, the kit for treating and/or preventing chronic skin diseases or the method for treating and/or preventing chronic skin diseases of the present invention. The PDE-IV inhibitor of the present invention includes all possible isomers and the mixtures thereof including the above.

To obtain salts of the PDE-IV inhibitor used in the present invention and the PDE-IV inhibitor of the present invention, when each compound is obtained in the form of a salt, it may be purified as it is. When each compound is obtained in the free form, it may be dissolved or suspended in a suitable solvent, followed by addition of an acid or a base to form a salt. Then, the resulting salt may be isolated and purified.

Furthermore, the PDE-IV inhibitor used in the present invention, the PDE-IV inhibitor of the present invention and pharmaceutically acceptable salts thereof may exist in the form of adducts with water or various solvents. These adducts can also be used as the pharmaceutical composition, the therapeutic and/or preventive agent for chronic skin diseases, the kit, the kit for treating and/or preventing chronic skin diseases or the method for treating and/or preventing chronic skin diseases of the present invention and they may be included in the PDE-IV inhibitor or a pharmaceutically acceptable salt thereof of the present invention.

As the immunosuppressant, any immunosuppressant can be used so long as it inhibits factors caused by inflammatory response such as cytokines or the number of mast cells and eosinophils, and suppresses the migration or the activation of inflammatory cells. Examples thereof include, Tacrolimus (the following Formula (A)), Pimecrolimus (the following Formula (B)), Ascomycin (the following Formula (C)), Rapamycin (the following Formula (D)), FTY 720 (the following Formula (E)), Azathioprine (the following Formula (F)), Cyclophosphamide (the following Formula (G)), Mizoribin (the following Formula (H)), Methotrexate (the following Formula (I)), Cyclosporin A (the following Formula (J)), Mofetil mycophenolate (the following Formula (K)), Brequinar sodium, Deoxyspergualin, and the like. Among them, Tacrolimus, Pimecrolimus, Ascomycin, Rapamycin, FTY 720, Azathioprine, Cyclophosphamide, Mizoribin, Methotrexate, Cyclosporin A and the like are preferred. These may be used alone or in combination.

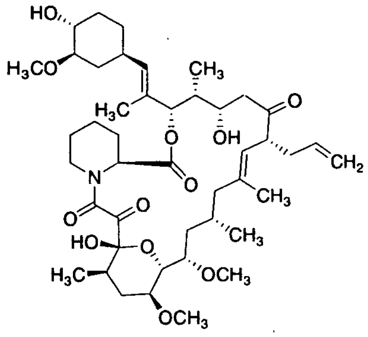
(A)

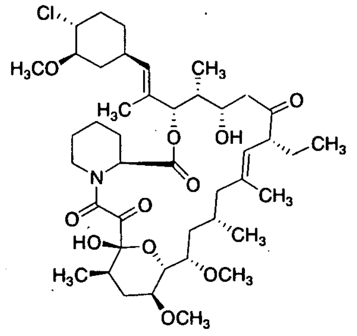
(B)

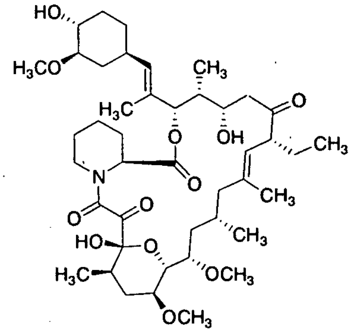
(C)

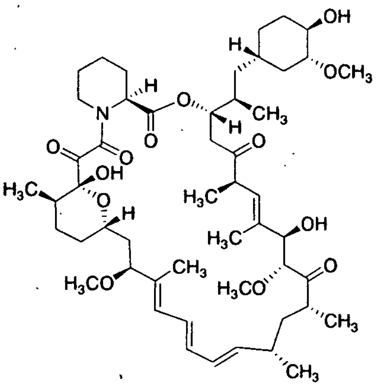
(D)

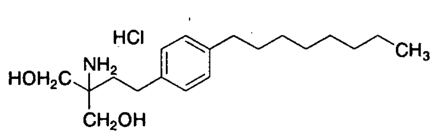
(E)

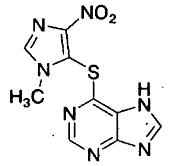
(F)

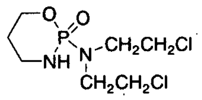
(G)

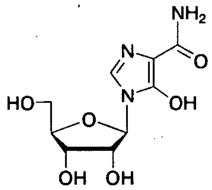
(H)

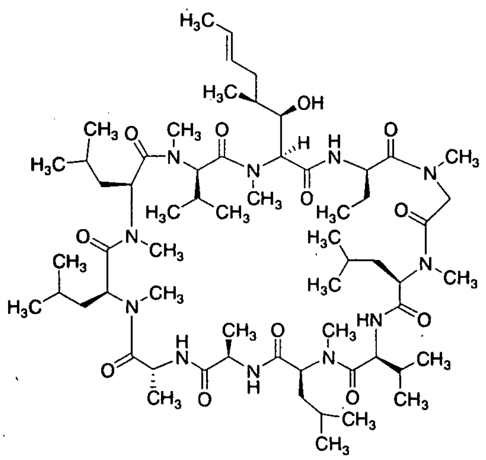
(J)

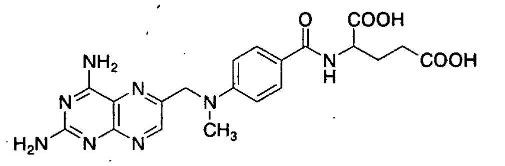
(I)

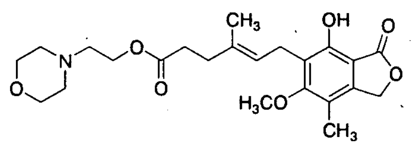
(K)

These immunosuppressants may exist as pharmaceutically acceptable salts (examples of the pharmaceutically acceptable salts include salts described as the pharmaceutically acceptable salts of the PDE-IV inhibitor described above and the like) or hydrates thereof. These pharmaceutically acceptable salts and hydrates can also be used for the pharmaceutical composition, the therapeutic and/or preventive agent for chronic skin diseases, the kit, the kit for treating and/or preventing chronic skin diseases, and the method for treating and/or preventing chronic skin diseases of the present invention. Also, each of the immunosuppressants may contain one or more asymmetric carbon(s) and two or more stereoisomers. However, all possible isomers including these and mixtures thereof can be used for the pharmaceutical composition, the therapeutic and/or preventive agent for chronic skin diseases, the kit, the kit for treating and/or preventing chronic skin diseases, and the method for treating and/or preventing chronic skin diseases of the present invention.

The immunosuppressants as described above can be obtained as commercially available products or by producing according to a conventionally known method.

The pharmaceutical composition and the therapeutic and/or preventive agent for chronic skin diseases comprising the PDE-IV inhibitor or a pharmaceutically acceptable salt thereof and the immunosuppressant of the present invention can be used for treating and/or preventing chronic skin diseases. For example, they can be used for treating and/or preventing chronic skin diseases such as contact dermatitis, atopic dermatitis, seborrheic dermatitis, nummular eczema, lichen simplex chronicus Vidal, autosensitization dermatitis, stasis dermatitis, asteatotic eczema, and psoriasis.

The PDE-IV inhibitor or a pharmaceutically acceptable salt thereof and the immunosuppressant used for the therapeutic and/or preventive agent for chronic skin diseases of the present invention can be used or administered as a monotherapy (drug combination) or as a combination of preparations as long as the preparations are prepared so as to contain each of these active ingredients. In particular, a combination of two or more preparations is preferred. When using or administering a combination of preparations, these preparations may be used or administered simultaneously or separately with an interval. The preparations are preferably used as a form such as a tablet, an injection or an external preparation, and in particular, an external preparation is preferred.

The dose ratio (weight/weight) of the PDE-IV inhibitor or a pharmaceutically acceptable salt thereof to the immunosuppressant may be appropriately adjusted according to the combination of the PDE-IV inhibitor with the immunosuppressant used, the efficacies of the PDE-IV inhibitor and the immunosuppressant, and the like. Specific example of the dose ratio is 1/50 (the PDE-IV inhibitor or a pharmaceutically acceptable salt thereof/the immunosuppressant) to 50000/1, preferably 1/30 to 10000/1, ' more preferably 1/20 to 5000/1., and further more preferably 1/10 to 1000/1.

When a combination of preparations is administered, for example, (a) a first component comprising the PDE-IV inhibitor or a pharmaceutically acceptable salt thereof and (b) a second component comprising the immunosuppressant are separately prepared as preparations respectively thereby to prepare a kit. And then; the kit can be used for administering the respective components to the same subject simultaneously or separately with an interval through the same route or different routes.

The kit may be formed of two or more vessels (for example, vials, bags and the like) and the contents (an active ingredient and the like), wherein the materials and the shape of the vessel is not particularly limited as long as it does not cause denaturation of the contents due to external temperature or light during preservation, or elution of the chemical component from the vessel. And the kit may be used as the form that enables the administration of the above first and second components of the contents through different routes (for example, tubes) or the same route. Specific examples thereof include a kit such as a tablet, an injection, an external preparation or the like.

The method for treating and/or preventing chronic skin diseases of the present invention can be performed-by the same method as the method for using or administering the PDE-IV inhibitor or a pharmaceutically acceptable salt thereof and the immunosuppressant which are used for the above-mentioned pharmaceutical composition or the therapeutic and/or preventive agent for chronic skin diseases. That is, the PDE-IV inhibitor or a pharmaceutically acceptable salt thereof and the immunosuppressant are prepared in a preparation so as to contain each of the active ingredients, and administered, for example, as a single preparation or as a combination of preparations, preferably as a combination of two or more preparations. When a combination of preparations is administered, the preparations can be administered simultaneously or separately with an interval or administered using a kit as described above.

Next, therapeutic effects on chronic skin diseases by simultaneously administering the PDE-IV inhibitor or a pharmaceutically acceptable salt thereof and the immunosuppressant are specifically described with the following Test Examples.

Test Example 1: The inhibitory effects on oxazolone-induced ear swelling in mice

Six-week-old BALB/c mice (male, Charles River Japan, Inc.) which are purchased are used for the experiment. After at least one week of quarantine and taming, seven-week-old mice which normally increased in weight and does not apparently show any abnormality are subjected to the experiment. The mice are placed in plastic cages (6 mice per cage) in a breeding room which is kept at a room temperature (19 to 25°C) and a humidity of 30 to 70% and is illuminated for 12 hours a day (from 7:00 a.m.,to 7:00 p.m.). The mice are allowed to freely intake commercially available pellets and water.

As an antigen solution, Oxazolone (Sigma-Aldrich) is dissolved in acetone (Kanto Kagaku) to prepare 0.5 w/v % of oxazolone-acetone solution. BALB/c mice are each applied with 100 μL of the antigen solution to the shaved abdomen for sensitization. Mice are shaved on abdomen on the previous day of the sensitization. The reaction is induced by an epicutaneous application of the antigen solution (10 μL) on the inner side of the ear 5 days after the sensitization. The test compound (the PDE-IV inhibitor or a pharmaceutically acceptable salt thereof and/or the immunosuppressant) is prepared by dissolving in acetone to make the concentration to the desired concentration (test compound solution). The test compound solution (10μL each, total of 20 μL) is applied to inner side and outer side of the ear 3 hours before and 2 hours after inducing the reaction. This group is referred to as a test compound administration group. The group

in which sensitization and inducing reaction are carried out and applied with the acetone 3 hours before and 2 hours after inducing the reaction is referred to as a positive control group, and the group which is unsensitized but the reaction is induced and applied with the acetone 3 hours before and 2 hours after inducing the reaction is referred to as a negative control group. Ear thickness is measured with a dial thickness gauge (Ozaki Seisakusho) just before and 24 hours after the application for inducing the reaction, and the difference in the thickness is used as an indication of ear swelling. The inhibition ratio (%) against ear swelling is calculated using the following:

Inhibition ratio (%) = [{(value in positive control group) - (value in test compound administration group)}/{(value in positive control group) - (value in negative control group)}] ×100

The above results show that the simultaneous administration of the PDE-IV inhibitor or a pharmaceutically acceptable salt thereof and the immunosuppressant has a therapeutic effect on chronic skin diseases.

Test Example 2: The inhibitory effects on oxazolone-induced ear swelling in mice

Six-week-old BALB/c mice (male, Charles River Japan, Inc.) were purchased. After one week of quarantine and taming, mice which steadily increased in weight and did not apparently show any abnormality were subjected to the experiment. The mice were placed in plastic cages (6 mice per cage) in a breeding room which was kept at a room temperature (19 to 25°C) and a humidity of 30 to 70% and was illuminated for 12 hours a day (from 7:00 a.m. to 7:00 p.m.). The mice were allowed to freely intake commercially available pellets and water.

As an antigen solution, Oxazolone (Sigma-Aldrich) was dissolved in acetone (Kanto Kagaku) to prepare 0.5 w/v % of oxazolone-acetone solution. BALB/c mice were each applied with 100 μL of the antigen solution to the shaved abdomen for sensitization. Mice were shaved on abdomen on the previous day of the sensitization. The reaction was induced by an epicutaneous application of the antigen solution (10 μL) on the inner side of the ear 5 days after the sensitization. Compound (I) and Tacrolimus were prepared by dissolving in acetone to make the concentration to 10 mg/mL and 0.01 mg/mL, respectively (Compound (I) solution, Tacrolimus solution). Compound (I) solution (10μL each, total of 20 μL) was applied to inner side and outer side of the ear 3 hours before and 2 hours after inducing the reaction (Compound (I) administration group). Similarly, Tacrolimus solution (10μL each, total of 20 μL) was applied to inner side and outer side of the ear 3 hours before and 2 hours after inducing the reaction (Tacrolimus administration group). Further, 20 μL of solution in which Compound (I) and prednisolone were dissolved in acetone to make the concentration to 10 mg/mL and 0.01 mg/mL, respectively, was similarly applied to inner side and outer side of the ear (combined administration group). The group in which sensitization and inducing reaction were carried out and applied with the acetone 3 hours before and 2 hours after inducing the reaction was referred to as a positive control group, and the group which was unsensitized but the reaction was induced and applied with the acetone 3 hours before and 2 hours after inducing the reaction was referred to as a negative control group. Ear thickness was measured with a dial thickness gauge (Ozaki Seisakusho) just before and 24 hours after inducing the reaction, and the difference in the thickness was used as an indication of ear swelling. The inhibition ratio (%) against ear swelling was calculated using the following:

Inhibition ratio (%) = [{(value in positive control group) - (value in test compound administration group)}/{(value in positive control group) - (value in negative control group)}] ×100

Test compound administration group: Compound (I) administration group, Tacrolimus administration group or combined administration group

The results are shown in Table 1.

Table 1

| Group | Dose (μg/site) | Inhibition Ratio |
|---|---|---|
| Compound (I) | 400 | 34%*** |
| Tacrolimus | 0.4 | 36%*** |
| Combined | Compound (1) 400 Tacrolimus 0.4 | 68%###, +++ |

***: P<0.001 (Student's t-test, compared to the positive control group)
###: P<0.05 (Student's t-test, compared to the Compound (I) administration group)
+++: P<0.001 (Student's t-test, compared to the Tacrolimus administration group)

In the Compound (I) administration group and the Tacrolimus administration group, a significant inhibition effect on ear swelling was observed, and the inhibition ratios were 34% (P<0.001) and 36% (P<0.001), respectively. Further, in the combined administration group of Compound (I) and Tacrolimus, the inhibition ratio was 68%, and significant inhibition was exhibited compared with the Compound (I) administration group and the Tacrolimus administration group.

Therefore, it was confirmed that by the combined administration of Compound (I) and Tacrolimus, namely, by the combined administration of the PDE-IV inhibitor and the immunosuppressant, a superior therapeutic effect on chronic skin diseases can be observed compared with the cases where the each agents is administered alone.

On the other hand, the immunosuppressant exhibits a strong antiinflammatory effect and is a good medicine as a therapeutic agent for chronic skin diseases. However, it has been reported that the immunosuppressant has a side effect such as carcinogenicity (Cancer, Vol. 80, p. 1141 (1997)).

The results of the above test show that the dose of the immunosuppressant conventionally used for treating chronic skin diseases can be decreased by using Compound (I) or a pharmaceutically acceptable salt thereof and the immunosuppressant in combination. In other words, the dose of the immunosuppressant conventionally used for treating chronic skin diseases can be decreased by the pharmaceutical composition, the, therapeutic and/or preventive agent for chronic skin diseases, the kit, the kit for treating and/or preventing chronic skin diseases or the method for treating and/or preventing chronic skin diseases of the present invention, and it is expected that the effect of the immunosuppressant used as a single preparation can be improved, and the above side effect can be also decreased.

As described above, the therapeutic and/or preventive agent for chronic skin diseases of the present invention can be used, administered or produced as a single preparation or a combination of preparations as long as they are prepared so as to contain the respective active ingredients of the PDE-IV inhibitor or a pharmaceutically acceptable salt thereof and the immunosuppressant. These pharmaceutical compositions or the therapeutic and/or preventive agents for chronic skin diseases are preferably in a unit dose form suitable for oral administration such as a tablet, or parenteral administration such as an injection or an external preparation. When a combination of preparations is used or administered, the preparations can be administered simultaneously or separately with an interval.

These preparations can be prepared by an ordinary method properly using the respective active ingredients and a pharmaceutically acceptable diluent, excipient, disintegrant, lubricant, binder, surfactant, water, physiological saline, vegetal oil solubilizer, isotonizing agent, preservative, antioxidant or the like.

In order to prepare a tablet, for example, an excipient such as lactose, a disintegrant such as starch, a lubricant such as magnesium stearate, a binder such as hydroxypropyl cellulose, a surfactant such as a fatty acid ester, a plasticizer such as glycerin, an antiseptic such as benzoic acid or the like may be used according to an ordinary method.

In order to prepare an injection, for example, water, physiological saline, a vegetal oil such as soybean oil, any of various solvents, a solubilizer, an isotonizing agent, a preservative, an antioxidant or the like may be used according to an ordinary method.

Suitable dosage forms for the external preparations, but not limited to, include preparations that are formed into cream, paste, jelly, gel, emulsion, liquid, or the like by dissolving or mixing and dispersing the active ingredient in base (e.g. ointments, liniments, lotions or the like); preparations that are formed by dissolving or mixing and dispersing the active ingredient and percutaneous absorption promoters in base, and then spreading them on supporting materials such as polyethylene, polyester, polyethylene terephthalate and the like (e.g. cataplasms, tapes or the like); and the like. Examples of above base include any pharmaceutically acceptable base, and known bases such as ointments, liniments, lotions and the like can be used. Examples of such base include sodium alginate; polymers such as gelatin, corn starch, tragacanth gum, methylcellulose, hydroxyethyl cellulose, carboxymethyl cellulose, xanthan gum, dextrin, carboxymethyl starch, polyvinyl alcohol, sodium polyacrylate, methoxyethylene-maleic anhydride copolymer, polyvinyl ether, polyvinyl pyrrolidone and the like; fats and oils such as yellow beeswax, olive oil, cacao oil, sesame oil, soybean oil, camellia oil, peanut oil, beef tallow, lard, lanolin and the like; vaseline such as white vaseline, yellow vaseline and

the like; paraffin; hydrocarbon gel ointments [for example, Plastibase[trade name(manufactured by Taisho Pharmaceutical Co., Ltd.)]; higher fatty acids such as stearic acid and the like; higher alcohols such as cetyl alcohol, stearyl alcohol and the like; polyethylene glycol; water and the like. Examples of above percutaneous absorption promoter include any pharmaceutically acceptable percutaneous absorption promoter, for example, alcohols such as methanol, ethanol, diethylene glycol, propylene glycol and the like; polar solvents such as dimethyl sulfoxide, dodecyl pyrrolidone and the like; urea; esters such as ethyl laurate, isopropyl myristate, cetyl octanoate and the like; azone; olive oil and the like. Additionally, inorganic fillers such as kaolin, bentonite, zinc oxide, titanium oxide and the like; viscosity-controlling agents; anti-aging agents; pH-controlling agents; humectants such as glycerin, propylene glycol and the like; and the like may be added to the base, if necessary.

Furthermore, the above external preparations may also contain diluents, flavors, and one or more additives selected from excipients, disintegrants, lubricants, binders, surfactants, plasticizers, antiseptics and the like, which are exemplified in the oral administration.

When using or administering the PDE-IV inhibitor or a pharmaceutically acceptable salt thereof and the immunosuppressant as a combination of various preparations, the dosages and the dosage frequencies may vary with the effects of each active ingredients, the dosage forms, age, weight and symptom of patients, and the like. Generally, the PDE-IV inhibitor or a pharmaceutically acceptable salt thereof and the immunosuppressant are preferably administered in a dose described below.

In the oral administration, such as tablets, the PDE-IV inhibitor or a pharmaceutically acceptable salt thereof and the immunosuppressant in a dose of 0.01 mg to 1000 mg and 0.01 to 1000 mg, respectively, preferably, 0.05 to 300 mg, and 0.1 to 300'mg, respectively, more preferably 0.5 to 200 mg, and 0.5 to 200 mg, respectively are administered to an adult patient once or several times a day simultaneously or separately with an interval.

In the parenteral administration, such as injection, the PDE-IV inhibitor or a pharmaceutically acceptable salt thereof and the immunosuppressant in a dose of 1 μg to 100 mg and 0.01 to 1000 mg, respectively, preferably, 5 μg to 30 mg and 0.05 to 100 mg, respectively, more preferably 10 μg to 20 mg, and 0.1 to 10 mg, respectively are administered to an adult patient once or several times a day simultaneously or separately with an interval.

The external preparation (e.g. ointment, cream or the like) generally contains 1 to 1000 mg, preferably, 3 to 300 mg, of the PDE-IV inhibitor or a pharmaceutically acceptable salt thereof and 1 to 1000 mg, preferably, 3 to 300 mg, of the immunosuppressant in 1 g of paste and is generally administered by applying it once or several times a day.

When using or administering the PDE-IV inhibitor or a pharmaceutically acceptable salt thereof and the immunosuppressant as a single preparation, the dosages and, the dosage frequencies may vary with the effects of active ingredients, the dosage forms, age, weight and symptom of patients, and the like. They are preferably used or administered as a single preparation prepared in a dose when used or administered as a combination of the above several preparations, respectively.

However, these dosages and frequencies vary based on the above-mentioned various conditions.

The embodiments of the present invention will now be described with following Examples but the scope of the present invention is not limited to these Examples.

Example 1: Tablet (Compound (I))

A tablet including the following composition is prepared by a conventional process. Compound (I) (40 g), lactose (286.8 g) and potato starch (60 g) are mixed, followed by adding 10% hydroxypropylcellulose aqueous solution (120 g) thereto. After the resulting mixture is kneaded, granulated, and dried according to a conventional process, the size of the granules is prepared for tablet pressing. The granules are mixed with magnesium stearate (1.2 g) and then pressed to make tablets (each tablet containing 20 mg of the active ingredient) by a tablet making machine having a striker of 8 mm diameter (Kikusui Co., Type RT-15).

| | | |
|---|---|---|
| Proscription | Compound (I) | 20 mg |
| | Lactose | 143.4 mg |
| | Potato starch | 30 mg |
| | Hydroxypropylcellulose | 6 mg |
| | Magnesium stearate | 0.6 mg |
| | | 200 mg |

Example 2: Tablet (Compound (III))

A tablet including the following composition is prepared by a conventional process. Compound (III) (40 g),

lactose (286.8 g) and potato starch (60 g) are mixed, followed by adding 10% hydroxypropylcellulose aqueous solution (120 g) thereto. After the resulting mixture is kneaded, granulated, and dried according to a conventional process, the size of the granules is prepared for tablet pressing. The granules are mixed with magnesium stearate (1.2 g) and then pressed to make tablets (each tablet containing 20 mg of the active ingredient) by a tablet making machine having a striker of 8 mm diameter (Kikusui Co., Type RT-15).

| | | |
|---|---|---|
| Proscription | Compound (III) | 20 mg |
| | Lactose | 143.4 mg |
| | Potato starch | 30 mg |
| | Hydroxypropylcellulose | 6 mg |
| | Magnesium stearate | 0.6 mg |
| | | 200 mg |

Example 3: Tablet (Compound (V))

A tablet including the following composition is prepared by a conventional process. Compound (V) (40 g), lactose (286.8 g) and,potato starch (60 g) are mixed, followed by adding 10% hydroxypropylcellulose aqueous solution (120 g) thereto. After the resulting mixture is kneaded, granulated, and dried according to a conventional process, the size of the granules is prepared for tablet pressing. The granules are mixed with magnesium stearate (1.2 g) and then pressed to make tablets (each tablet containing 20 mg of the active ingredient) by a tablet making machine having a striker of 8 mm diameter (Kikusui Co., Type RT-15).

| | | |
|---|---|---|
| Proscription | Compound (V) | 20 mg |
| | Lactose | 143.4 mg |
| | Potato starch | 30 mg |
| | Hydroxypropylcellulose | 6 mg |
| | Magnesium stearate | 0.6 mg |
| | | 200 mg |

Example 4: Tablet (Tacrolimus)

A tablet including the following composition is prepared by a conventional process. Tacrolimus (40 g), lactose (286.8 g) and potato starch (60 g) are mixed, followed by adding 10% hydroxypropylcellulose aqueous solution (120 g) thereto. After the resulting mixture is kneaded, granulated, and dried according to a conventional process, the size of the granules is prepared for tablet pressing. The granules are mixed with magnesium stearate (1.2 g) and then pressed to make tablets (each tablet containing 20 mg of the active ingredient) by a tablet making machine having a striker of 8 mm diameter (Kikusui Co., Type RT-15).

| | | |
|---|---|---|
| Proscription | Tacrolimus | 20 mg |
| | Lactose | 143.4 mg |
| | Potato starch | 30 mg |
| | Hydroxypropylcellulose | 6 mg |
| | Magnesium stearate | 0.6 mg |
| | | 200 mg |

Example 5: Tablet (A monotherapy of Compound (I) and Tacrolimus)

A tablet including the following composition is prepared by a conventional process. Compound (I) (40 g), Tacrolimus (40 g), lactose (246.8 g) and potato-starch (40 g) are mixed, followed by adding 10% hydroxypropylcellulose aqueous solution (120 g) thereto. After the resulting mixture is kneaded, granulated, and dried according to a conventional process, the size of the granules is prepared for tablet pressing. The granules are mixed with magnesium stearate (1.2 g) and then pressed to make tablets (each tablet containing 20 mg of Compound (I) and 20 mg of Tacrolimus) by a tablet making machine having a striker of 8 mm diameter (Kikusui Co., Type RT-15).

| | | |
|---|---|---|
| Proscription | Compound (I) | 20 mg |
| | Tacrolimus | 20 mg |
| | Lactose | 123.4 mg |
| | Potato starch | 20 mg |
| | Hydroxypropylcellulose | 6 mg |
| | Magnesium stearate | 0.6 mg |
| | | 200 mg |

Example 6: Tablet (A monotherapy of Compound (III) and Cyclosporin A)

A tablet including the following composition is prepared by a conventional process. Compound (III) (40 g), Cyclosporin A (40 g), lactose (246.8 g) and potato starch (40 g) are mixed, followed by adding 10% hydroxypropylcellulose aqueous solution (120 g) thereto. After the resulting mixture is kneaded, granulated, and dried according to a conventional process, the size of the granules is prepared for tablet pressing. The granules are mixed with magnesium stearate (1.2 g) and then pressed to make tablets (each tablet containing 20 mg of Compound (III) and 20 mg of Cyclosporin A) by a tablet making machine having a striker of 8 mm diameter (Kikusui Co., Type RT-15).

| | | |
|---|---|---|
| Proscription | Compound (III) | 20 mg |
| | Cyclosporin A | 20 mg |
| | Lactose | 123.4 mg |
| | Potato starch | 20 mg |
| | Hydroxypropylcellulose | 6 mg |
| | Magnesium stearate | 0.6 mg |
| | | 200 mg |

Example 7: Injection (Compound (IV))

An injection including the following composition is prepared by a conventional process. Compound (IV) (1 g) is dissolved in purified soybean oil, and purified egg-yolk lecithin (12 g) and glycerin (25 g) for injection are added thereto. Injectable distilled water is added to the resulting mixture to make the total volume to 1000 mL, and the resulting mixture is kneaded and emulsified according to a conventional process. The resulting dispersion is filtered with a 0.2 μm disposable membrane filter under sterile condition and is dispensed into glass vials at a volume of 2 mL per vial (each vial contains 2 mg of the active ingredient) under the sterile condition to obtain the injections.

| | | |
|---|---|---|
| Prescription | Compound (IV) | 2 mg |
| | Purified soybean oil | 200 mg |
| | Purified egg-yolk lecithin | 24 mg |
| | Glycerin for injection | 50 mg |
| | Injectable distilled water | 1.72 mL |
| | | 2.00 mL |

Example 8: Injection (Cyclosporin A)

An injection including the following composition is prepared by a conventional process. Cyclosporin A (1 g) is dissolved in purified soybean oil, and purified egg-yolk lecithin (12 g) and glycerin (25 g) for injection are added thereto. Injectable distilled water is added to the resulting mixture to make the total volume to 1000 mL, and the resulting mixture is kneaded and emulsified according to a conventional process. The resulting dispersion is filtered with a 0.2 μm disposable membrane filter under sterile condition and is dispensed into glass vials at a volume of 2 mL per vial (each vial contains 2 mg of the active ingredient) under the sterile condition to obtain the injections.

| | | |
|---|---|---|
| Prescription | Cyclosporin A | 2 mg |
| | Purified soybean oil | 200 mg |

(continued)

| | |
|---|---|
| Purified egg-yolk lecithin | 24 mg |
| Glycerin for injection | 50 mg |
| Injectable distilled water | 1.72 mL |
| | 2.00 mL |

Example 9: Injection (A monotherapy of Compound (VI) and Pimecrolimus)

An injection including the following composition is prepared by a conventional process. Compound (VI) (1 g) and Pimecrolimus (1 g) is dissolved in purified soybean oil, and purified egg-yolk lecithin (12 g) and glycerin (25 g) for injection are added thereto. Injectable distilled water is added to the resulting mixture to make the total volume to 1000 mL, and the resulting mixture is kneaded and emulsified according to a conventional process. The resulting dispersion is filtered with a 0.2 μm disposable membrane filter under sterile condition and is dispensed into glass vials at a volume of 2 mL per vial (each vial contains 2 mg of the Compound (VI) and 2 mg of Pimecrolimus) under the sterile condition to obtain the injections.

| | | |
|---|---|---|
| Prescription | Compound (VI) | 2 mg |
| | Pimecrolimus | 2 mg |
| | Purified soybean oil | 200 mg |
| | Purified egg-yolk lecithin | 24 mg |
| | Glycerin for injection | 50 mg |
| | Injectable distilled water | 1.72 mL |
| | | 2.00 mL |

Example 10: External preparation (Compound (I))

An external preparation including the following composition is prepared according to a conventional process. White Vaseline (65 g) is heated with stirring, and propylene glycol (25 g) is added thereto. To the resulting mixture, a mixture of Compound (I) (5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (I) | 5 g |
| | White Vaseline | 65 g |
| | Propylene glycol | 25 g |
| | Cetyl octanoate | 5 g |
| | | 100 g |

Example 11: External preparation (Compound (II))

An external preparation including the following composition is prepared according to a conventional process. White Vaseline (65 g) is heated with stirring, and propylene glycol (25 g) is added thereto. To the resulting mixture, a mixture of Compound (II) (5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (II) | 5 g |
| | White Vaseline | 65 g |
| | Propylene glycol | 25 g |
| | Cetyl octanoate | 5 g |
| | | 100 g |

Example 12: External preparation (Compound (III))

An external preparation including the following composition is prepared according to a conventional process. White Vaseline (65 g) is heated with stirring, and propylene glycol (25 g) is added thereto. To the resulting mixture, a mixture of Compound (III) (5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (III) | 5 g |
| | White Vaseline | 65 g |
| | Propylene glycol | 25 g |
| | Cetyl octanoate | 5 g |
| | | 100 g |

Example 13: External preparation (Compound (IV))

An external preparation including the following composition is prepared according to a conventional process. White Vaseline (65 g) is heated with stirring, and propylene glycol (25 g) is added thereto. To the resulting mixture, a mixture of Compound (IV) (5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (IV) | 5 g |
| | White Vaseline | 65 g |
| | Propylene glycol | 25 g |
| | Cetyl octanoate | 5 g |
| | | 100 g |

Example 14: External preparation (Compound (VIII)')

An external preparation including the following composition is prepared according to a conventional process. White Vaseline (65 g) is heated with stirring, and propylene glycol (25 g) is added thereto. To the resulting mixture, a mixture of Compound (VIII) (5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (VIII) | 5 g |
| | White Vaseline | 65 g |
| | Propylene glycol | 25 g |
| | Cetyl octanoate | 5 g |
| | | 100 g |

Example 15: External preparation (Cyclosporin A)

An external preparation including the following composition is prepared according to a conventional process. White Vaseline (69.5 g) is heated with stirring, and propylene glycol (25 g) is added thereto. To the resulting mixture, a mixture of Cyclosporin A (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Cyclosporin A | 0.5 g |
| | White Vaseline | 69.5 g |
| | Propylene glycol | 25 g |
| | Cetyl octanoate | 5 g |
| | | 100 g |

Example 16: External preparation (FTY 720)

An external preparation including the following composition is prepared according to a conventional process. White Vaseline (69.5 g) is heated with stirring, and propylene glycol (25 g) is added thereto. To the resulting mixture, a mixture of FTY 720 (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | FTY 720 | 0.5 g |
| | White Vaseline | 69.5 g |
| | Propylene glycol | 25 g |
| | Cetyl octanoate | 5 g |
| | | 100 g |

Example 17: External preparation (Tacrolimus)

An external preparation including the following composition is prepared according to a conventional process. White Vaseline (69.5 g) is heated with stirring, and propylene glycol (25 g) is added thereto. To the resulting mixture, a mixture of Tacrolimus (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Tacrolimus | 0.5 g |
| | White Vaseline | 69.5 g |
| | Propylene glycol | 25 g |
| | Cetyl octanoate | 5 g |
| | | 100 g |

Example 18: External preparation (A monotherapy of Compound (I) and Cyclosporin A)

An external preparation including the following composition is prepared according to a conventional process. White Vaseline (64.5 g) is heated with stirring, and propylene glycol (25 g) is added thereto. To the resulting mixture, a mixture of Compound (I) (5 g), Cyclosporin A (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (I) | 5 g |
| | Cyclosporin A | 0.5 g |
| | White Vaseline | 64.5 g |
| | Propylene glycol | 25 g |
| | Cetyl octanoate | 5 g |
| | | 100 g |

Example 19: External preparation (A monotherapy of Compound (III) and Cyclosporin A)

An external preparation including the following composition is prepared according to a conventional process. White Vaseline (64.5 g) is heated with stirring, and propylene glycol (25 g) is added thereto. To the resulting mixture, a mixture of Compound (III) (5 g), Cyclosporin A (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (III) | 5 g |
| | Cyclosporin A | 0.5 g |
| | White Vaseline | 64.5 g |
| | Propylene glycol | 25 g |

(continued)

| | | |
|---|---|---|
| | Cetyl octanoate | 5 g |
| | | 100 g |

Example 20: External preparation (A monotherapy of Compound (I) and Tacrolimus)

An external preparation including the following composition is prepared according to a conventional process. White Vaseline (64.5 g) is heated with stirring, and propylene glycol (25 g) is added thereto. To the resulting mixture, a mixture of Compound (I) (5 g), Tacrolimus (0.5 g) and cetyl octanoate (5 g) is added and dispersed with continuous stirring and heating. Then, the dispersion is gradually cooled to about 25°C and put' into an appropriate vessel to obtain the external ointment.

| | | |
|---|---|---|
| Prescription | Compound (I) | 5 g |
| | Tacrolimus | 0.5 g |
| | White Vaseline | 64.5 g |
| | Propylene glycol | 25 g |
| | Cetyl octanoate | 5 g |
| | | 100 g |

Example 21: External preparation (A monotherapy of Compound (III) and Tacrolimus)

An external preparation including the following composition is prepared according to a conventional process. White Vaseline (64.5 g) is heated with stirring, and propylene glycol (25 g) is added thereto. To the resulting mixture, a mixture of Compound (III) (5 g), Tacrolimus (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (III) | 5 g |
| | Tacrolimus | 0.5 g |
| | White Vaseline | 64.5 g |
| | Propylene glycol | 25 g |
| | Cetyl octanoate | 5 g |
| | | 100 g |

Example 22: External preparation (A monotherapy of Compound (V) and Tacrolimus)

An external preparation including the following composition is prepared according to a conventional process. White Vaseline (64.5 g) is heated with stirring, and propylene glycol (25 g) is added thereto. To the resulting mixture, a mixture of Compound (V) (5 g), Tacrolimus (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (V) | 5 g |
| | Tacrolimus | 0.5 g |
| | White Vaseline | 64.5 g |
| | Propylene glycol | 25 g |
| | Cetyl octanoate | 5 g |
| | | 100 g |

Example 23: External preparation (A monotherapy of Compound (VII) and Tacrolimus)

An external preparation including the following composition is prepared according to a conventional process.

White Vaseline (64.5 g) is heated with stirring, and propylene glycol (25 g) is added thereto. To the resulting mixture, a mixture of Compound (VII) (5. g), Tacrolimus (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (VII) | 5 g |
| | Tacrolimus | 0.5 g |
| | White Vaseline | 64.5 g |
| | Propylene glycol | 25 g |
| | Cetyl octanoate | 5 g |
| | | 100 g |

Example 24: External preparation (A monotherapy of Compound (I) and FTY 720)

An external preparation including the following composition is prepared according to a conventional process. White Vaseline (64.5 g) is heated with stirring, and propylene glycol (25 g) is added thereto. To the resulting mixture, a mixture of Compound (I) (5 g), FTY 720 (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (I) | 5 g |
| | FTY 720 | 0.5 g |
| | White Vaseline | 64.5 g |
| | Propylene glycol | 25 g |
| | Cetyl octanoate | 5 g |
| | | 100 g |

Example 25: External preparation (A monotherapy of Compound (VIII) and FTY 720)

An external preparation including the following composition is prepared according to a conventional process. White Vaseline (64.5 g) is heated with stirring, and propylene glycol (25 g) is added thereto. To the resulting mixture, a mixture of Compound (VIII) (5 g), FTY 720 (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (VIII) | 5 g |
| | FTY 720 | 0.5 g |
| | White Vaseline | 64.5 g |
| | Propylene glycol | 25 g |
| | Cetyl octanoate | 5 g |
| | | 100 g |

Example 26: External preparation (A monotherapy of Compound (I) and Pimecrolimus)

An external preparation including the following composition is prepared according to a conventional process. White Vaseline (64.5 g) is heated with stirring, and propylene glycol (25 g) is added thereto. To the resulting mixture, a mixture of Compound (I) (5 g), Pimecrolimus (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (I) | 5 g |
| | Pimecrolimus | 0.5 g |
| | White Vaseline | 64.5 g |

(continued)

| | |
|---|---|
| Propylene glycol | 25 g |
| Cetyl octanoate | 5 g |
| | 100 g |

Example 27: External preparation (A monotherapy of Compound (II) and Pimecrolimus)

An external preparation including the following composition is prepared according to a conventional process. White Vaseline (64.5 g) is heated with stirring, and propylene glycol (25 g) is added thereto. To the resulting mixture, a mixture of Compound (II) (5 g), Pimecrolimus (0.5 g) and cetyl octanoate (5 g) is added and dispersed with continuous stirring and heating. Then, the dispersion is gradually cooled to about 25°C and put into an appropriate vessel to obtain the external ointment.

| | | |
|---|---|---|
| Prescription | Compound (II) | 5 g |
| | Pimecrolimus | 0.5 g |
| | White Vaseline | 64.5 g |
| | Propylene glycol | 25 g |
| | Cetyl octanoate | 5 g |
| | | 100 g |

Industrial Applicability

The present invention provides a pharmaceutical composition comprising (a) PDE-IV inhibitor or a pharmaceutically acceptable salt thereof and (b) immunosuppressant, a therapeutic and/or preventive agent for chronic skin diseases comprising (a) PDE-IV inhibitor or a pharmaceutically acceptable salt thereof and (b) immunosuppressant, and the like.

## Claims

1. A pharmaceutical composition which comprises (a) a phosphodiesterase (PDE)-IV inhibitor or a pharmaceutically acceptable salt thereof and (b) an immunosuppressant.

2. The pharmaceutical composition according to Claim 1, wherein the PDE-IV inhibitor is a compound selected from a group consisting of compounds represented by Formulae (I) to (XIV):

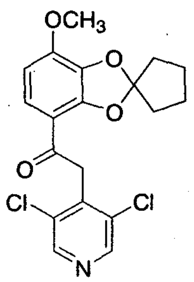

( I )

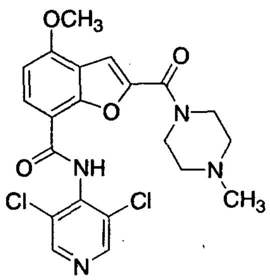

( II )

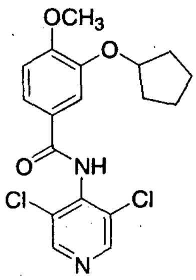

( III )

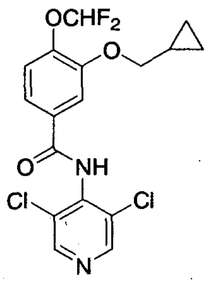

( IV )

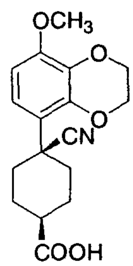

( V )

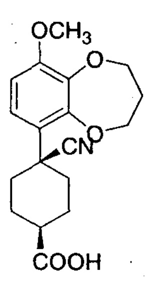
( VI )

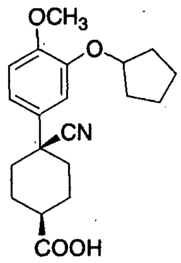
( VII )

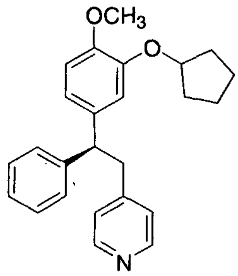
( VIII )

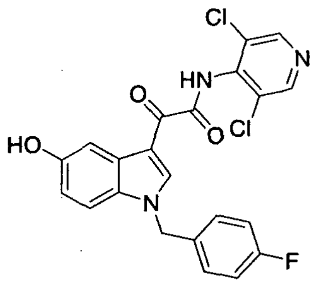
( IX )

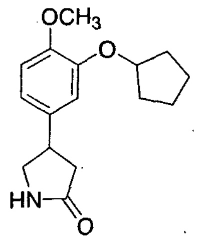
( X )

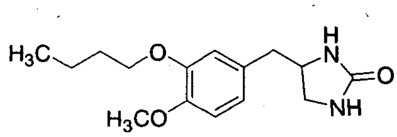
( XI )

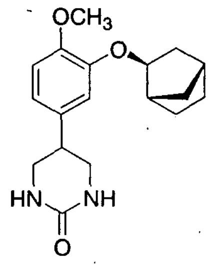
( XII )

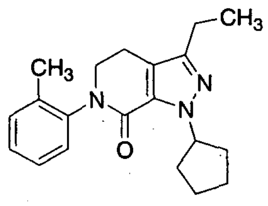
( XIII )

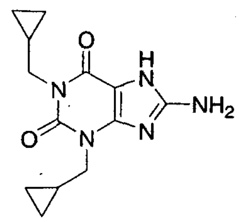
( XIV )

3. The pharmaceutical composition according to Claim 1, wherein the PDE-IV inhibitor is a compound represented by Formula (I):

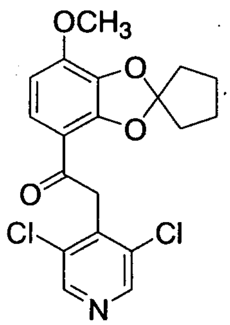
( I )

4. The pharmaceutical composition according to any one of Claims 1 to 3, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

5. A therapeutic and/or preventive agent for chronic skin diseases which comprises (a) a PDE-IV inhibitor or a pharmaceutically acceptable salt thereof and (b) an immunosuppressant, as active ingredients.

6. The therapeutic and/or preventive agent for chronic skin diseases according to Claim 5, wherein the PDE-IV inhibitor is a compound selected from a group consisting of compounds represented by Formulae (I) to (XIV):

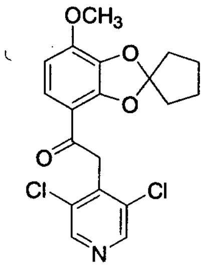
( I )

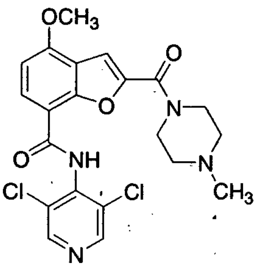
( II )

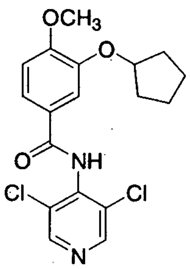
( III )

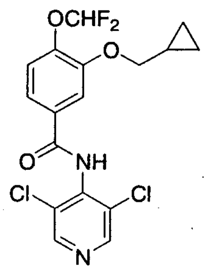
( IV )

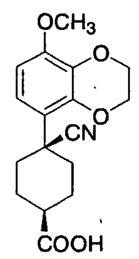
( V )

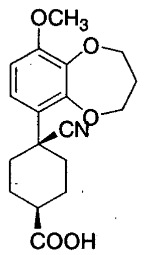
( VI )

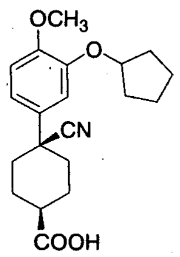
( VII )

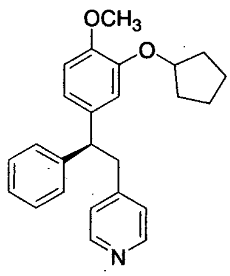
( VIII )

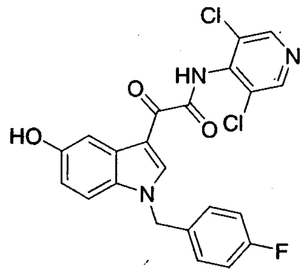
( IX )

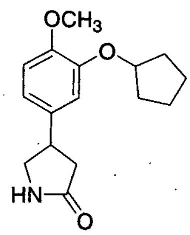
( X )

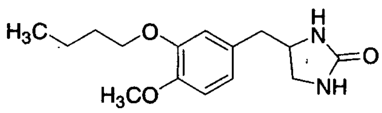
( XI )

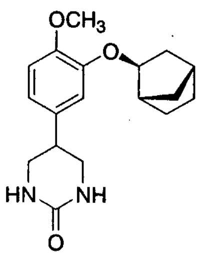
( XII )

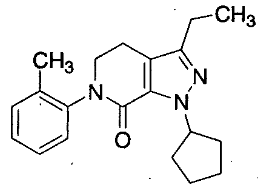
( XIII )

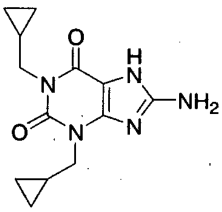
( XIV )

7. The therapeutic and/or preventive agent for chronic skin diseases according to Claim 5, wherein the PDE-IV inhibitor is a compound represented by Formula (I):

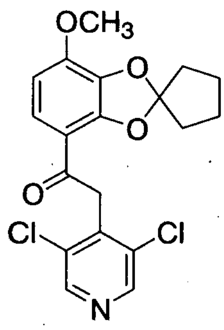
( I )

8. The therapeutic and/or preventive agent for chronic skin diseases according to any one of Claims 5 to 7, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

**9.** The therapeutic and/or preventive agent for chronic skin diseases according to any one of Claims 5 to 8, wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

**10.** The therapeutic and/or preventive agent for chronic skin diseases according to any one of Claims 5 to 9, wherein the agent is an external preparation.

**11.** A therapeutic and/or preventive agent for chronic skin diseases for administering simultaneously or separately with an interval, which comprises (a) a PDE-IV inhibitor or a pharmaceutically acceptable salt thereof and (b) an immunosuppressant, as active ingredients.

**12.** The therapeutic and/or preventive agent for chronic skin diseases according to Claim 11, wherein the PDE-IV inhibitor is a compound selected from a group consisting of compounds represented by Formulae (I) to (XIV):

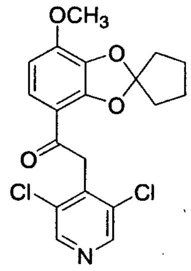
( I )

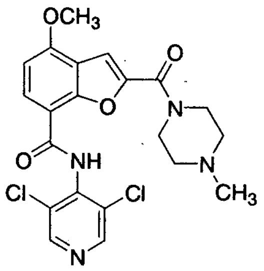
( II )

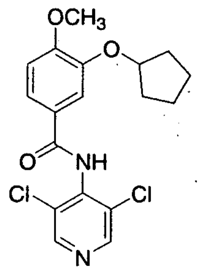
( III )

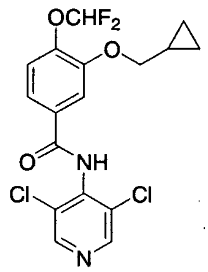
( IV )

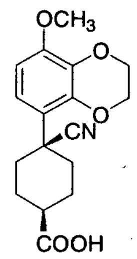
( V )

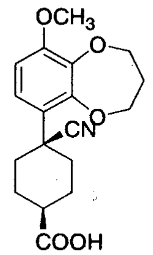
( VI )

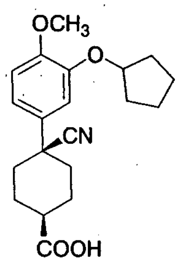
( VII )

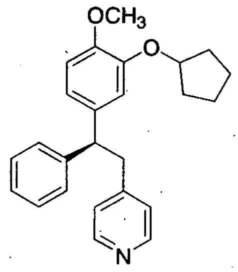
( VIII )

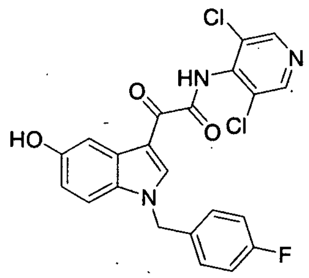
( IX )

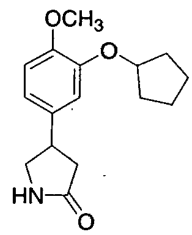
( X )

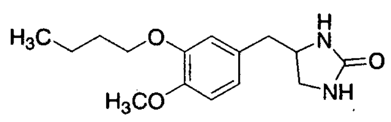
( XI )

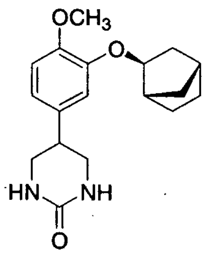
( XII )

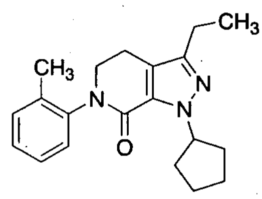
( XIII )

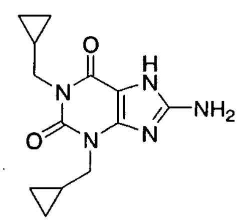
( XIV )

**13.** The therapeutic and/or preventive agent for chronic skin diseases according to Claim 11, wherein the-PDE-IV inhibitor is a compound represented by Formula (I):

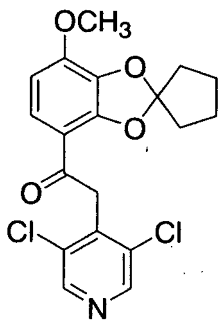

( I )

**14.** The therapeutic and/or preventive agent for chronic skin diseases according to any one of Claims 11 to 13, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

**15.** The therapeutic and/or preventive agent for chronic skin diseases according to any one of Claims 11 to 14, wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

**16.** The therapeutic and/or preventive agent for chronic skin diseases according to any one of Claims 11 to 15, wherein the agent is an external preparation.

**17.** A kit **characterized by** comprising (a) a first component comprising a PDE-IV inhibitor or a pharmaceutically acceptable salt thereof and (b) a second component comprising an immunosuppressant.

**18.** The kit according to Claim 17, wherein the PDE-IV inhibitor is a compound selected from a group consisting of compounds represented by Formulae (I) to (XIV):

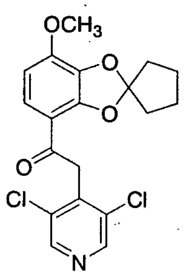

( I )

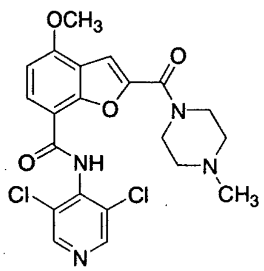

( II )

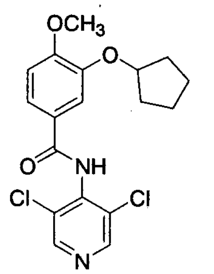

( III )

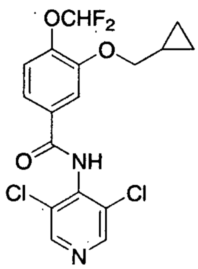

( IV )

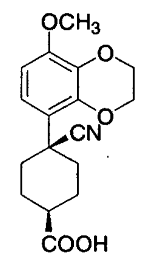

( V )

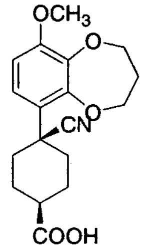

( VI )

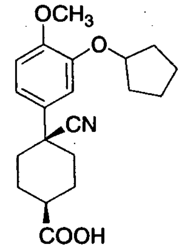

( VII )

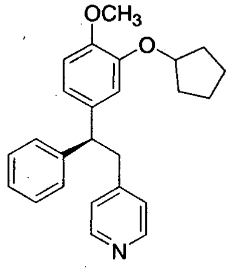

( VIII )

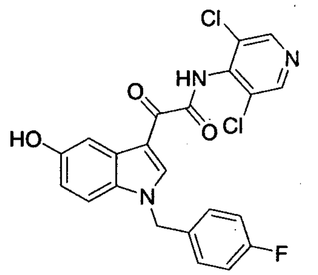

( IX )

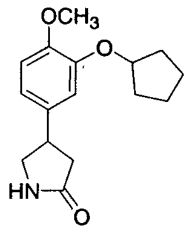

( X )

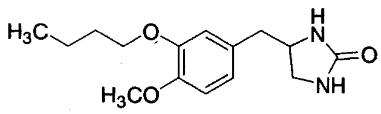
( XI )

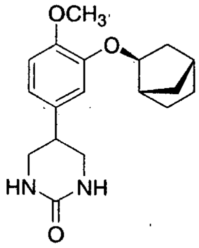
( XII )

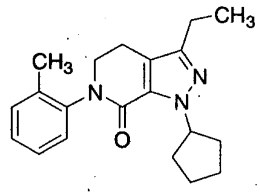
( XIII )

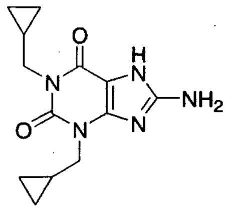
( XIV )

**19.** The kit according to Claim 17, wherein the PDE-IV inhibitor is a compound represented by Formula (I):

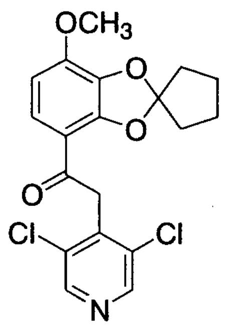
( I )

**20.** The kit according to any one of Claims 17 to 19, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

**21.** A kit for treating and/or preventing chronic skin diseases **characterized by** comprising (a) a first component comprising a PDE-IV inhibitor or a pharmaceutically acceptable salt thereof and (b) a second component comprising an immunosuppressant.

**22.** The kit for treating and/or preventing chronic skin diseases according to Claim 21, wherein the PDE-IV inhibitor is a compound selected from a group consisting of compounds represented by Formulae (I) to (XIV):

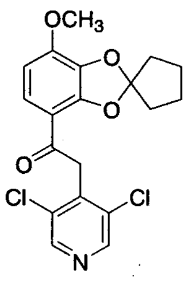
( I )

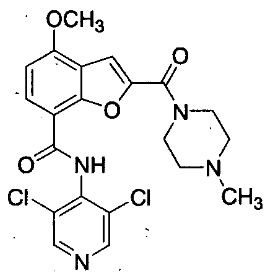
( II )

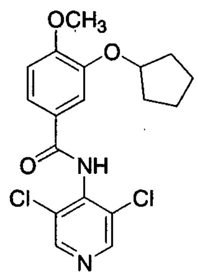
( III )

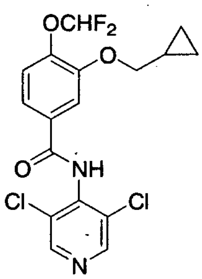
( IV )

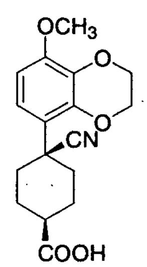
( V )

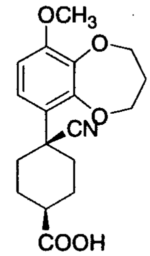

( VI )

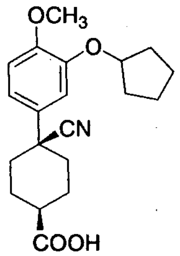

( VII )

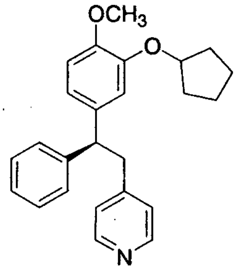

( VIII )

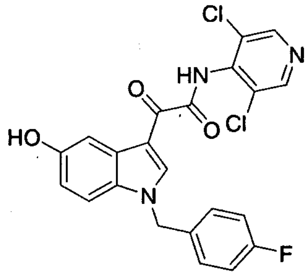

( IX )

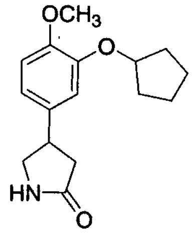

( X )

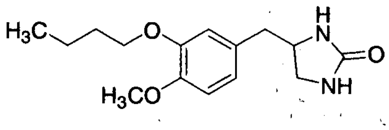

( XI )

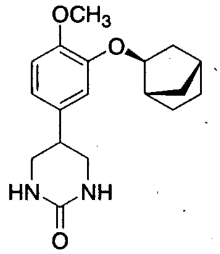

( XII )

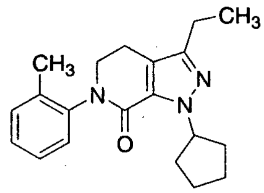

( XIII )

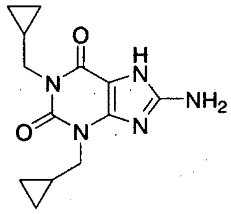

( XIV )

**23.** The kit for treating and/or preventing chronic skin diseases according to Claim 21, wherein the PDE-IV inhibitor is a compound represented by Formula (I):

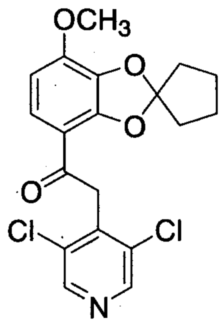

( I )

**24.** The kit for treating and/or preventing chronic skin diseases according to any one of Claims 21 to 23, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

**25.** The kit for treating and/or preventing chronic skin diseases according to any one of Claims 21 to 24, wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

**26.** The kit for treating and/or preventing chronic skin diseases according to any one of Claims 21 to 25, wherein the kit is a kit of external preparations.

**27.** A PDE-IV inhibitor or a pharmaceutically acceptable salt thereof for administering simultaneously or separately with an interval with an immunosuppressant.

**28.** The PDE-IV inhibitor or a pharmaceutically acceptable salt thereof according to Claim 27, wherein the PDE-IV inhibitor is a compound selected from a group consisting of compounds represented by Formulae (I) to (XIV):

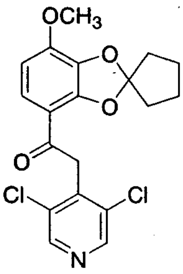
( I )

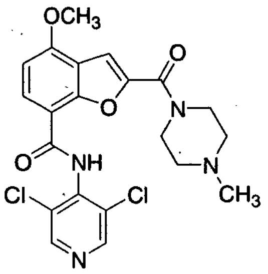
( II )

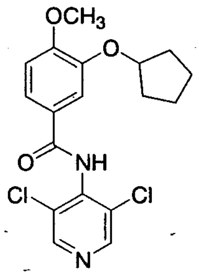
( III )

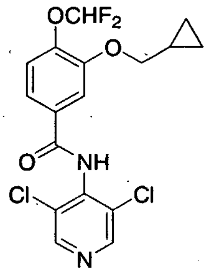
( IV )

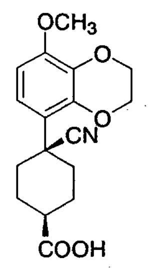
( V )

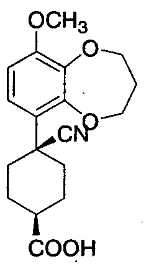
( VI )

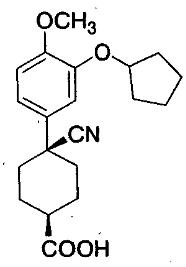
( VII )

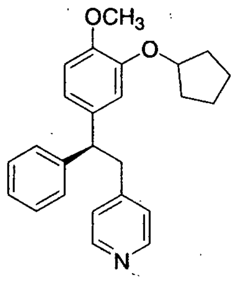
( VIII )

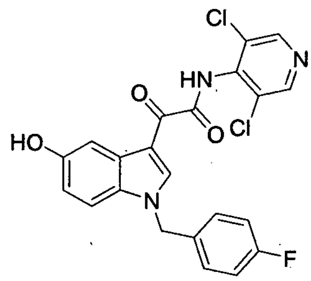
( IX )

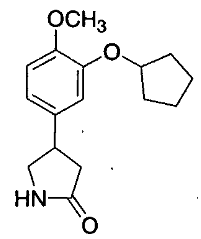
( X )

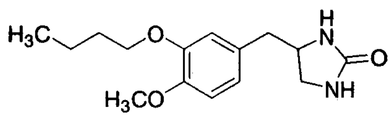
( XI )

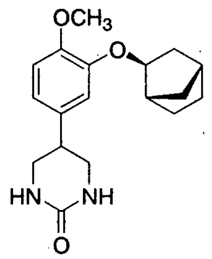
( XII )

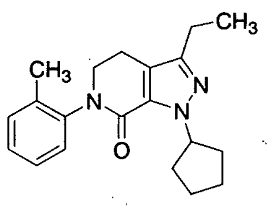
( XIII )

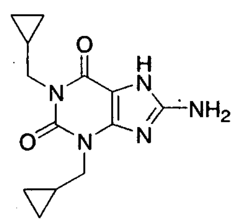
( XIV )

**29.** The PDE-IV inhibitor or a pharmaceutically acceptable salt thereof according to Claim 27, wherein the PDE-IV inhibitor is a compound represented by Formula (I):

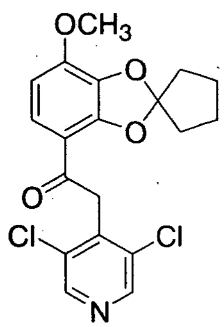
( I )

**30.** The PDE-IV inhibitor or a pharmaceutically acceptable salt thereof according to any one of Claims 27 to 29, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

**31.** A pharmaceutical composition which comprises, as an active ingredient, a PDE-IV inhibitor or a pharmaceutically acceptable salt thereof for administering simultaneously or separately with an interval with an immunosuppressant.

**32.** The pharmaceutical composition according to Claim 31, wherein the PDE-IV inhibitor is a compound selected from a group consisting of compounds represented by Formulae (I) to (XIV):

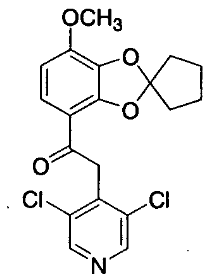
( I )

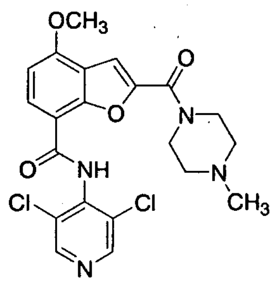
( II )

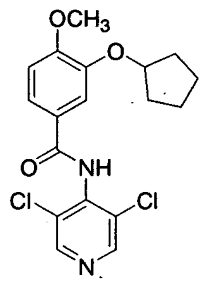
( III )

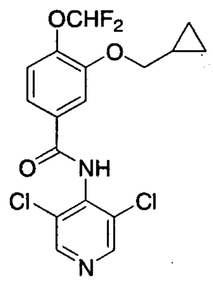
( IV )

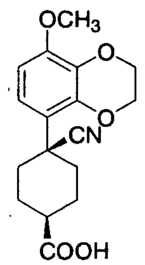
( V )

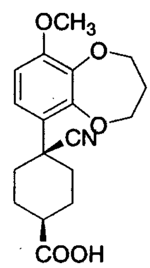
( VI )

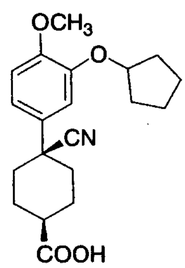
( VII )

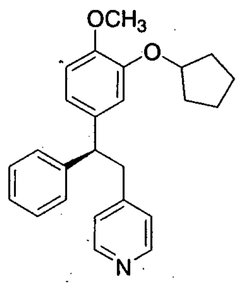
( VIII )

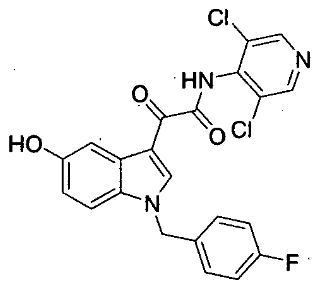
( IX )

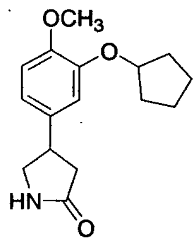
( X )

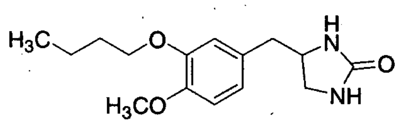
( XI )

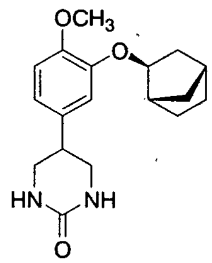
( XII )

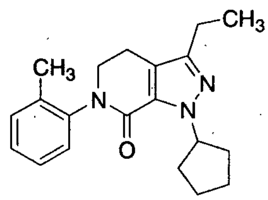
( XIII )

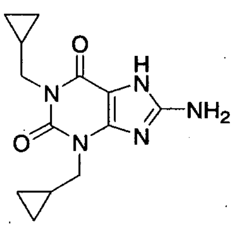
( XIV )

**33.** The pharmaceutical composition according to Claim 31, wherein the PDE-IV inhibitor is a compound represented by Formula (I):

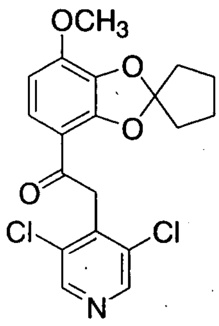

( I )

**34.** The pharmaceutical composition according to any one of Claims 31 to 33, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

**35.** A method for treating and/or preventing chronic skin diseases **characterized by** administering (a) a PDE-IV inhibitor or a pharmaceutically acceptable salt thereof and (b) an immunosuppressant simultaneously or separately with an interval.

**36.** The method for treating and/or preventing chronic skin diseases according to Claim 35, wherein the PDE-IV inhibitor is a compound selected from a group consisting of compounds represented by Formulae (I) to (XIV):

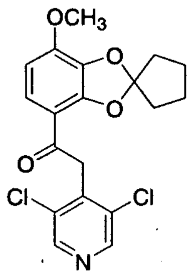

( I )

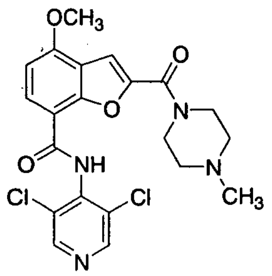

( II )

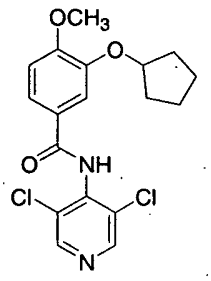

( III )

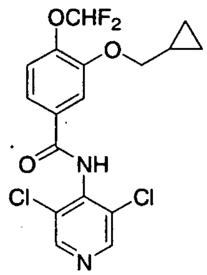

( IV )

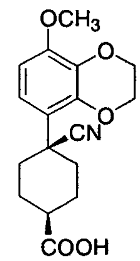

( V )

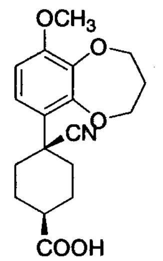

( VI )

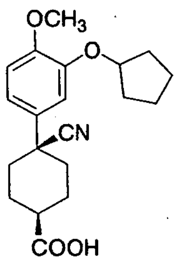

( VII )

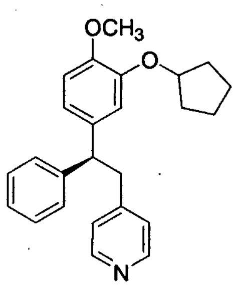

( VIII )

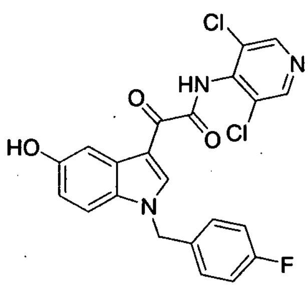

( IX )

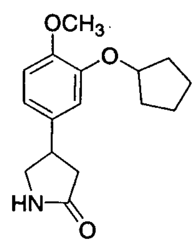

( X )

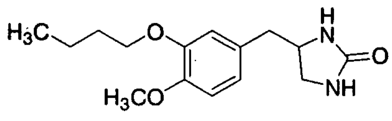

( XI )

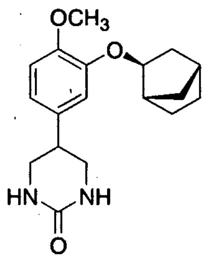

( XII )

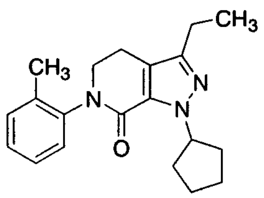

( XIII )

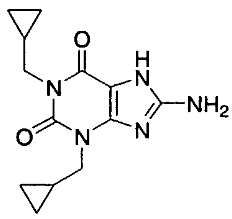

( XIV )

**37.** The method for treating and/or preventing chronic skin diseases according to Claim 35, wherein the PDE-IV inhibitor is a compound represented by Formula (I):

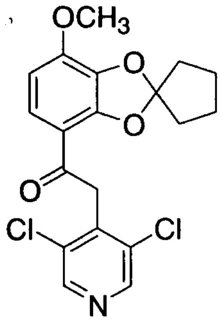

( I )

**38.** The method for treating and/or preventing chronic skin diseases according to any one of Claims 35 to 37, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

**39.** The method for treating and/or preventing chronic skin diseases according to any one of Claims 35 to 38, wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

**40.** The method for treating and/or preventing chronic skin diseases according to any one of Claims 35 to 39, **characterized by** administering (a) a PDE-IV inhibitor or a pharmaceutically acceptable salt thereof and (b) an immunosuppressant as external preparations.

**41.** A method for treating and/or preventing chronic skin diseases **characterized by** administering an effective amount of a combination of (a) a PDE-IV inhibitor or a pharmaceutically acceptable salt thereof and (b) an immunosuppressant.

**42.** The method for treating and/or preventing chronic skin diseases according to Claim 41, wherein the PDE-IV inhibitor is a compound selected from a group consisting of compounds represented by Formulae (I) to (XIV):

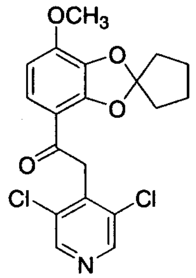
( I )

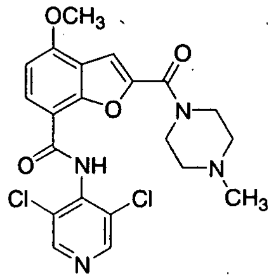
( II )

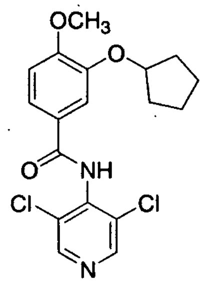
( III )

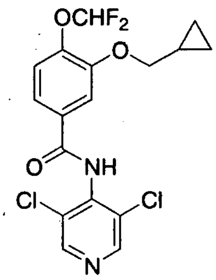
( IV )

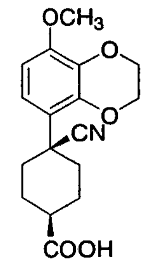
( V )

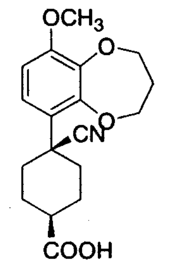
( VI )

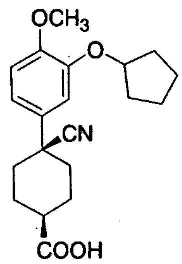
( VII )

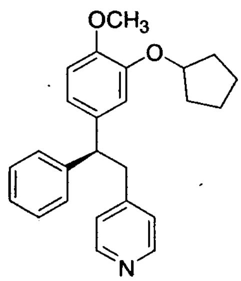
( VIII )

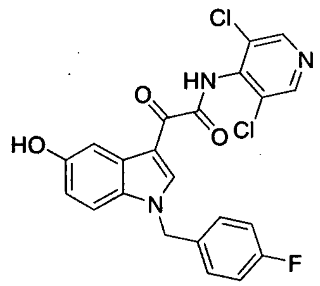
( IX )

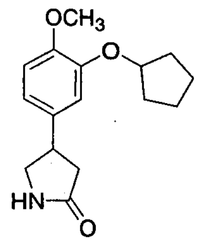
( X )

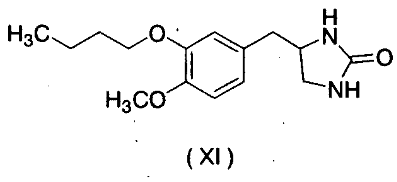
( XI )

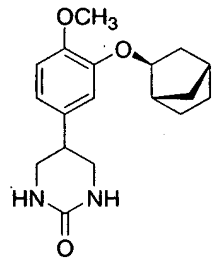
( XII )

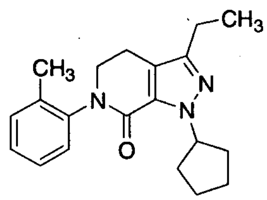
( XIII )

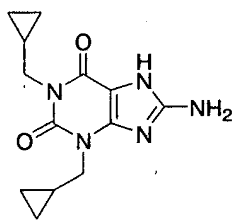
( XIV )

**43.** The method for treating and/or preventing chronic skin diseases according to Claim 41, wherein the PDE-IV inhibitor is a compound represented by Formula (I):

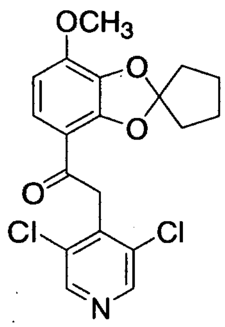
( I )

**44.** The method for treating and/or preventing chronic skin diseases according to any one of Claims 41 to 43, wherein the immunosuppressant is an immunosuppressant selected from a group consisting of Tacrolimus, Pimecrolimus,

Ascomycin, Rapamycin, FTY 720, Azathioprine, Cyclophosphamide, Methotrexate, Mizoribine and Cyclosporin A.

**45.** The method for treating and/or preventing chronic skin diseases according to any one of Claims 41 to 44, wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

**46.** The method for treating and/or preventing chronic skin diseases according to any one of Claims 41 to 45, wherein the administrating is an administration of external preparations.

**47.** Use of (a) a PDE-IV inhibitor or a pharmaceutically acceptable salt thereof and (b) an immunosuppressant for the manufacture of a therapeutic and/or preventive agent for chronic skin diseases.

**48.** Use according to Claim 47, wherein the PDE-IV inhibitor is a compound selected from a group consisting of compounds represented by Formulae (I) to (XIV):

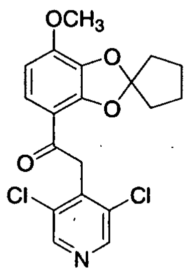
( I )

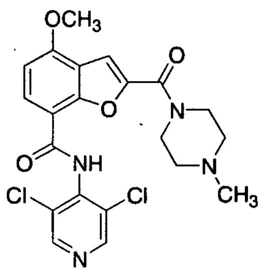
( II )

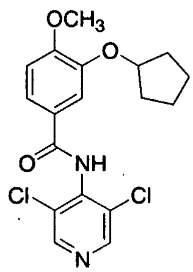
( III )

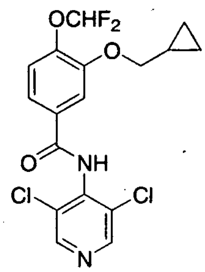
( IV )

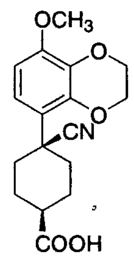
( V )

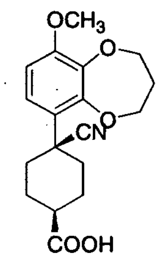
( VI )

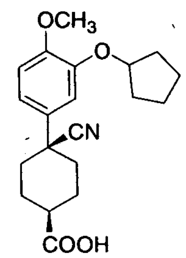
( VII )

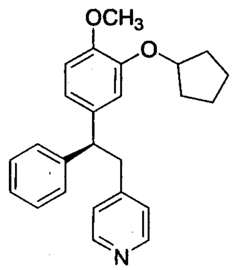
( VIII )

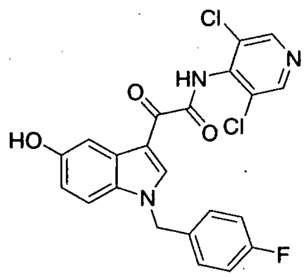
( IX )

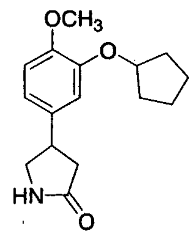
( X )

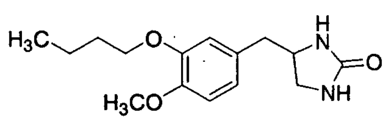
( XI )

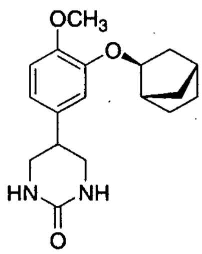
( XII )

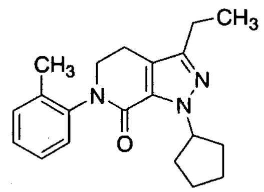
( XIII )

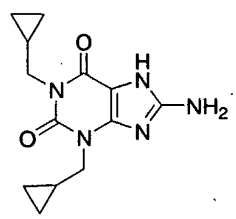
( XIV )

**49.** Use according to Claim 47, wherein the PDE-IV inhibitor is a compound represented by Formula (I):

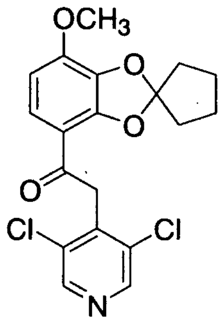

( I )

**50.** Use according to any one of Claims 47 to 49, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

**51.** Use according to any one of Claims 47 to 50, wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

**52.** Use according to any one of Claims 47 to 51, wherein the therapeutic and/or preventive agent for chronic skin diseases is an external preparation.

**53.** Use of (a) a PDE-IV inhibitor or a pharmaceutically acceptable salt thereof and (b) an immunosuppressant for the manufacture of a therapeutic and/or preventive agent for chronic skin diseases to be administered simultaneously or separately with an interval, which comprises (a) and (b), as active ingredients.

**54.** Use according to Claim 53, wherein the PDE-IV inhibitor is a compound selected from a group consisting of compounds represented by Formulae (I) to (XIV):

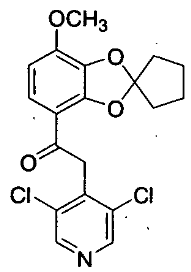

( I )

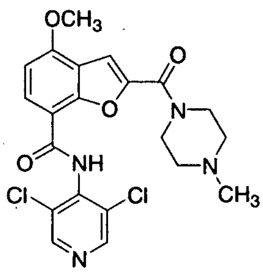

( II )

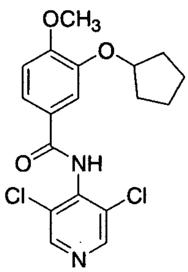

( III )

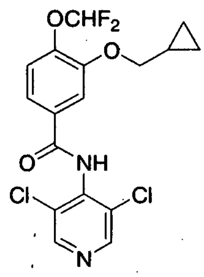

( IV )

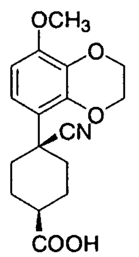

( V )

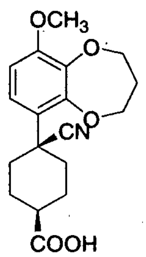

( VI )

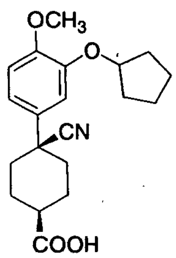

( VII )

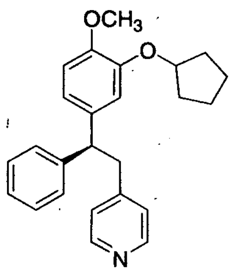

( VIII )

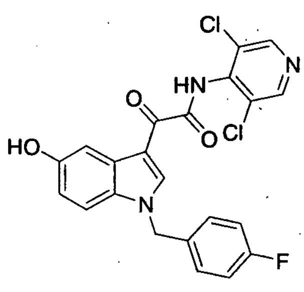

( IX )

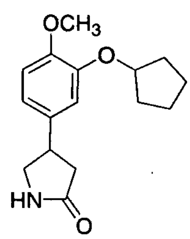

( X )

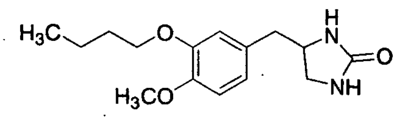

( XI )

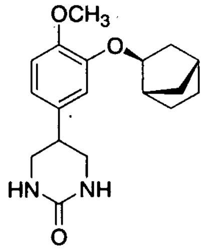

( XII )

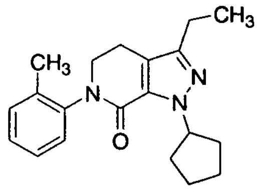

( XIII )

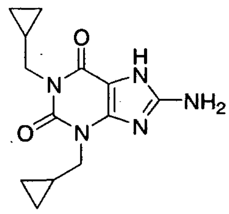

( XIV )

**55.** Use according to Claim 53, wherein the PDE-IV inhibitor is a compound represented by Formula (I):

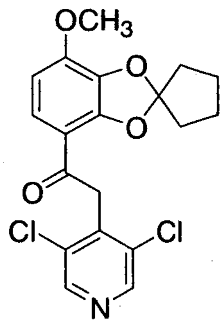

( I )

**56.** Use according to any one of Claims 53 to 55, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

**57.** Use according to any one of Claims 53 to 56, wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

**58.** Use according to any one of Claims 53 to 57, wherein the therapeutic and/or preventive agent for chronic skin diseases is an external preparation.

**59.** Use of (a) a PDE-IV inhibitor or a pharmaceutically acceptable salt thereof and (b) an immunosuppressant, for the manufacture of a kit for treating and/or preventing chronic skin diseases which comprises a first component comprising (a) and a second component comprising (b).

**60.** Use according to Claim 59, wherein the PDE-IV inhibitor is a compound selected from a group consisting of compounds represented by Formulae (I) to (XIV):

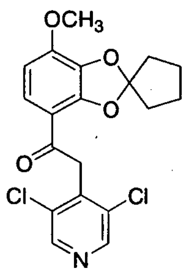

( I )

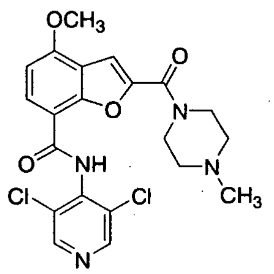

( II )

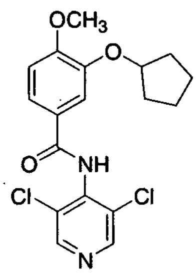

( III )

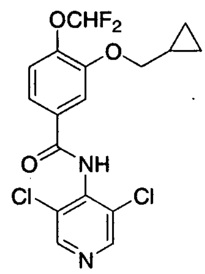

( IV )

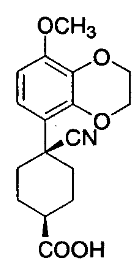

( V )

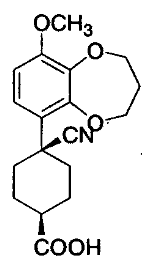

( VI )

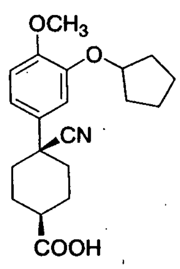

( VII )

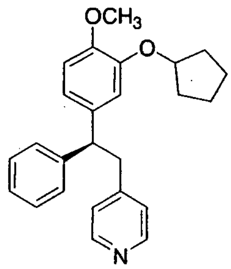

( VIII )

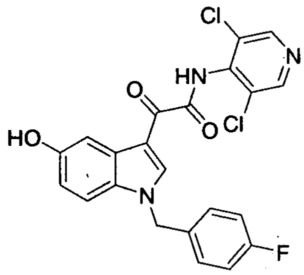

( IX )

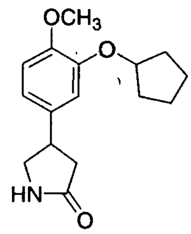

( X )

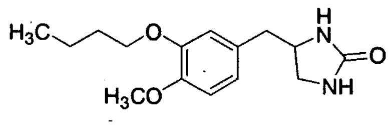

( XI )

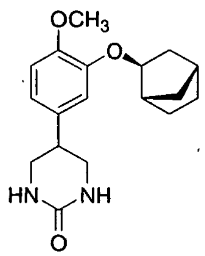

( XII )

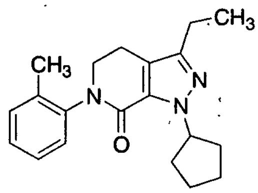

( XIII )

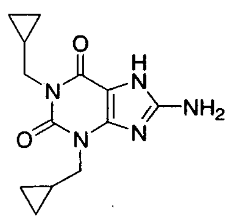

( XIV )

**61.** Use according to Claim 59, wherein the PDE-IV inhibitor is a compound represented by Formula (I):

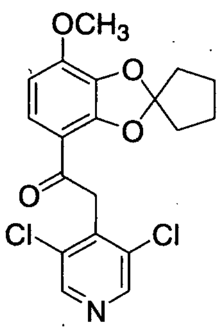

( I )

**62.** Use according to any one of Claims 59 to 61, wherein the immunosuppressant is an immunosuppressant selected from a group consisting of Tacrolimus, Pimecrolimus, Ascomycin, Rapamycin, FTY 720, Azathioprine, Cyclophosphamide, Methotrexate, Mizoribine and Cyclosporin A.

**63.** Use according to any one of Claims 59 to 62, wherein the chronic skin disease is a disease selected from a group consisting of contact dermatitis, atopic dermatitis, seborrheic dermatitis, nummular eczema, lichen simplex chronicus Vidal, autosensitization dermatitis, stasis dermatitis, asteatotic eczema, and psoriasis.

**64.** Use according to any one of Claims 59 to 63, wherein the kit for treating and/or preventing chronic skin diseases is a kit of external preparations.

**INTERNATIONAL SEARCH REPORT**

International application No.
PCT/JP2005/018854

A. CLASSIFICATION OF SUBJECT MATTER
***A61K45/06***(2006.01), ***A61K31/277***(2006.01), ***A61K31/357***(2006.01), ***A61K31/44***(2006.01), ***A61K31/4409***(2006.01), ***A61K31/443***(2006.01), ***A61K31/496***(2006.01), ***A61K31/706***(2006.01), ***A61K38/00***(2006.01), ***A61P17/00***(2006.01),
According to International Patent Classification (IPC) or to both national classification and IPC

B. FIELDS SEARCHED

Minimum documentation searched (classification system followed by classification symbols)
Int.Cl$^7$ A61K45/00-45/08, A61K31/00-31/80, A61K38/00-38/42, A61P1/00-43/00, C07D401/00-421/14, C07D213/00-213/75

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS(STN), CAplus(STN), EMBASE(STN), MEDLINE(STN), JMEDPlus(JOIS), JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| X<br><br><br><br><br>Y | WO 2004/058729 A1 (ALMIRALL PRODESFARMA SA), 15 July, 2004 (15.07.04), Claims; examples<br>& ES 2211344 A1 & EP 1575926 A1<br>& NO 200503614 A & AU 2003290110 A1<br>& BR 200316883 A | 1,4,5,8-11, 14-17,20,21, 24-27,30,31, 34,47,50-53, 56-59,62-64<br>3,6,7,12,13, 19,22,23,33, 48,49,54,55, 60,61 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 January, 2006 (13.01.06) | 24 January, 2006 (24.01.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2005/018854

C (Continuation). DOCUMENTS CONSIDERED TO BE RELEVANT

| Category* | Citation of document, with indication, where appropriate, of the relevant passages | Relevant to claim No. |
|---|---|---|
| X<br><br><br>Y | ROTH-EICHHORN, S. et al., Anti-Proliferative Properties of the Phosphodiesterase-4 inhibitor Rolipram Can Supplement Immunosuppressive Effects of Cyclosporine for Treatment of Obliterative Bronchiolitis in Heterotopic Rat Allografts. J. Heart Transplant., 2001, 20(11), pages 1188 to 1198, full text, particularly, paragraph of "METHODS", Fig. 1 | 1,2,4,17,18,20,21,27,28,30-32,34<br>3,6,7,12,13,19,22,23,33,48,49,54,55,60,61 |
| X<br><br><br>Y | BRIGGS, W.A. et al., Lymphocyte Suppression by Rolipram with Other Immunosuppressive Drugs. J.Clin.Pharmacol., 1999, 39, pages 794 to 799, full text, particularly, summary, tables 2, 3 | 1,2,4,17,18,20,21,27,28,30-32,34<br>3,6,7,12,13,19,22,23,33,48,49,54,55,60,61 |
| X<br>Y | BOGUNIEWICZ, M., Advances in the understanding and treatment of atopic dermatitis. Curr. Opin. Periatrics, 1997, 9(6), pages 577 to 581, full text, particularly, summary, paragraph of "Immunomodulatory therapy" | 27,28,30<br>3,6,7,12,13,19,22,23,33,48,49,54,55,60,61 |
| X<br>Y | SMITH, C.H., New approaches to topical therapy., Clin. Exp. Dermatol. 2000, 25(7), 567-574, full text, particularly, summary, page 572, right column, paragraph of "Phosphodiesterase inhibitors" | 27,30<br>3,6,7,12,13,19,22,23,33,48,49,54,55,60,61 |
| X<br>Y | DOHERTY, A.M., Phosphodiesterase 4 inhibitors as novel anti-nflammatory agents. Curr. Opin. Chem. Biol., 1999, 3, pages 466 to 473, paragraph of "Atopic eczema" | 27,28,30<br>3,6,7,12,13,19,22,23,33,48,49,54,55,60,61 |
| X<br>Y | BÄUMER, W. et al., Effects of the phosphodiesterase 4 inhibitors SB 207499 and AWD 12-281 on the inflammatory reaction in a model of a allergic dermatitis. Eur. J. Pharmacol., 2002, 446, pages 195 to 200, full text | 27,28,30<br>3,6,7,12,13,19,22,23,33,48,49,54,55,60,61 |
| X<br>Y | GRIFFITHS, C.E.M. et al., Randomized comparison of the type 4 phosphodiesterase inhibitor cipamfylline cream vehicle and hydrocortisone 17-butyrate cream for the treatment of atopic dermatitis. Br. J. Dermatol., 2002, 147, pages 299 to 307, full text | 27,28,30<br>3,6,7,12,13,19,22,23,33,48,49,54,55,60,61 |
| X<br>Y | HANIFIN, J.M. et al., Biochemical and immunologic mechanisms in atopic dermatitis: New targets for emerging therapies. J. Am. Acad. Dermatol., 1999, 41, pages 72 to 77, full text | 27,28,30<br>3,6,7,12,13,19,22,23,33,48,49,54,55,60,61 |

Form PCT/ISA/210 (continuation of second sheet) (April 2005)

**INTERNATIONAL SEARCH REPORT**

International application No.
PCT/JP2005/018854

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 96/36624 A1 (Kyowa Hakko Kogyo Co., Ltd.),<br>21 November, 1996 (21.11.96),<br>Full text; particularly, example 140<br>& AU 9657029 A & NO 9700151 A<br>& EP 771794 A1 & KR 97704724 A<br>& US 2002/0128290 A1 & US 6514996 B2<br>& CN 1154697 A & US 6716987 B1 | 27-30<br>3,6,7,12,13,<br>19,22,23,33,<br>48,49,54,55,<br>60,61 |
| X<br>Y | WO 99/16768 A1 (Kyowa Hakko Kogyo Co., Ltd.),<br>08 April, 1999 (08.04.99),<br>Particularly, examples<br>& AU 9892811 A & EP 1029860 A1<br>& US 6395738 B1 & US 6716987 B1 | 27,28,30<br>3,6,7,12,13,<br>19,22,23,33,<br>48,49,54,55,<br>60,61 |
| X<br>Y | JP 8-512041 A (BYK Gulden Lomberg Chemische<br>Fabrik GmbH.),<br>17 December, 1996 (17.12.96),<br>Particularly, examples<br>& WO 95/01338 A1 & AU 9474907 A<br>& FI 9506333 A & NO 9505211 A<br>& EP 706513 A1 & CZ 9600001 A3<br>& SK 9501617 A3 & CN 1126468 A<br>& NZ 271316 A & US 5712298 A<br>& BR 1100886 A3 | 27,28,30<br>3,6,7,12,13,<br>19,22,23,33,<br>48,49,54,55,<br>60,61 |
| X<br>Y | WO 00/14085 A1 (Kyowa Hakko Kogyo Co., Ltd.),<br>16 March, 2000 (16.03.00),<br>Particularly, examples<br>& AU 200022511 A & EP 1110961 A1<br>& BR 9913405 A & US 2001/0056117 A1<br>& CN 1317002 A & KR 2001073099 A<br>& US 6376535 B2 & HU 200103896 A2<br>& MX 2001002102 A1 | 27,28,30<br>3,6,7,12,13,<br>19,22,23,33,<br>48,49,54,55,<br>60,61 |
| X<br>Y | JP 7-504442 A (Celltech Ltd.),<br>18 May, 1995 (18.05.95),<br>Particularly, examples<br>& WO 94/14742 A1 & AU 9457092 A<br>& FI 9403856 A & EP 626939 A1<br>& ZA 9309623 A & NO 9403091 A<br>& GB 2279075 A & CZ 9402034 A3<br>& NZ 258982 A & CN 1092064 A<br>& US 5622977 A & BR 1100742 A3<br>& IL 108132 A | 27,28,30<br>3,6,7,12,13,<br>19,22,23,33,<br>48,49,54,55,<br>60,61 |

Form PCT/ISA/210 (continuation of second sheet) (April 2005)

**INTERNATIONAL SEARCH REPORT**

International application No.
PCT/JP2005/018854

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2002-513017 A (Arzneimittelwerk Dresden GmbH),<br>08 May, 2002 (08.05.02),<br>Particularly, examples<br>& WO 99/55696 A1 & AU 9938229 A<br>& DE 19917504 A1 & BR 9910029 A<br>& NO 200005454 A & EP 1076657 A1<br>& ZA 200005540 A & US 6251923 B1<br>& SK 200001575 A3 & CZ 200003985 A3<br>& KR 2001043106 A & CN 1307575 A<br>& HU 200101625 A2 & US 2002/0111351 A1<br>& US 2002/0115651 A1 & US 2002/0137745 A1<br>& US 2002/0119971 A1 & NZ 507406 A<br>& US 6545025 B2 & US 6545158 B2<br>& US 2003/0134876 A1 & US 6613794 B2<br>& EP 1475377 A1 & US 2004/0220183 A1 | 27,28,30<br>3,6,7,12,13,<br>19,22,23,33,<br>48,49,54,55,<br>60,61 |
| X<br>Y | JP 6-507405 A (SmithKline Beecham Corp.),<br>25 August, 1994 (25.08.94),<br>Particularly, examples<br>& WO 92/19594 A1 & AU 9219170 A<br>& ZA 9203210 A & PT 100441 A<br>& CN 1067244 A & EP 584208 A1 | 27,28,30<br>3,6,7,12,13,<br>19,22,23,33,<br>48,49,54,55,<br>60,61 |
| X<br>Y | GRISWOLD, D.E. et al., SB 207499 (Ariflo), a Second Generation Phosphodiesterase 4 Inhibitor, Reduces Tumor Necrosis Factor $\alpha$ and Interleukin-4 Production *in vivo*. J. Pharmacol. Exp. Ther., 1998, 287(2), pages 705 to 711, full text | 27,28,30<br>3,6,7,12,13,<br>19,22,23,33,<br>48,49,54,55,<br>60,61 |
| X<br>Y | ASHTON, M.J. et al., Selective Type IV Phosphodiesterase Inhibitors as Antiasthmatic Agents. The Syntheses and Biological Activities of 3-(Cyclopentyloxy)-4-methoxybenzamides and Analogues. J. Med. Chem., 1994, 37, pages 1696 to 1703, full text | 27,28,30<br>3,6,7,12,13,<br>19,22,23,33,<br>48,49,54,55,<br>60,61 |
| X<br>Y | CHRISTENSEN, S.B. et al., 1,4-Cyclohexanecarboxylates: Potent and Selective Inhibitors of Phosphodiesterase 4 for the Treatment of Asthma. J. Med. Chem., 1998, 41, pages 821 to 835, full text | 27,28,30<br>3,6,7,12,13,<br>19,22,23,33,<br>48,49,54,55,<br>60,61 |
| X<br>Y | DUPLANTIER, A.J. et al., 7-Oxo-4,5,6,7-tetrahydro-1*H*-pyrazolo[3,4-*c*]pyridines as Novel Inhibitors of Human Eosinophil Phosphodiesterase. J. Med. Chem., 1998, 41, pages 2268 to 2277, full text | 27,28,30<br>3,6,7,12,13,<br>19,22,23,33,<br>48,49,54,55,<br>60,61 |

Form PCT/ISA/210 (continuation of second sheet) (April 2005)

**INTERNATIONAL SEARCH REPORT**

International application No.
PCT/JP2005/018854

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 11-209350 A (Eisai Co., Ltd.),<br>03 August, 1999 (03.08.99),<br>Full text<br>& WO 99/37622 A1 & EP 1052254 A1<br>& US 6352989 B1 & US 2002/0055516 A1<br>& US 6800644 B2 | 27,30<br>1-26,28,29,<br>31-34,47-61 |
| X<br>A | JP 2000-504303 A (F. Hoffmann-La Roche AG.),<br>11 April, 2000 (11.04.00),<br>Full text<br>& WO 96/02541 A1 & US 5466697 A<br>& AU 9526818 A & EP 770078 A1 | 27,30<br>1-26,28,29,<br>31-34,47-61 |
| P,X<br><br><br><br><br>P,Y | WO 2005/049581 A1 (ALMIRALL PRODESFARMA, S.A.),<br>02 June, 2005 (02.06.05),<br>Claims; examples<br>& ES 2232306 A1 | 1,4,5,8-11,<br>14-17,20,21,<br>24-27,30,31,<br>34,47,50-53,<br>56-59,62-64<br>3,6,7,12,13,<br>19,22,23,33,<br>48,49,54,55,<br>60,61 |

Form PCT/ISA/210 (continuation of second sheet) (April 2005)

**INTERNATIONAL SEARCH REPORT**

International application No.
PCT/JP2005/018854

Continuation of A. CLASSIFICATION OF SUBJECT MATTER
(International Patent Classification (IPC))

***A61P17/06***(2006.01), ***A61K31/4433***(2006.01), ***A61K31/4427***(2006.01), ***A61K31/436***(2006.01), *C07D405/06*(2006.01), *C07D405/12*(2006.01), *C07D213/30*(2006.01), *C07D213/75*(2006.01)

(According to International Patent Classification (IPC) or to both national classification and IPC)

Form PCT/ISA/210 (extra sheet) (April 2005)

**INTERNATIONAL SEARCH REPORT**

International application No.
PCT/JP2005/018854

**Box No. II Observations where certain claims were found unsearchable (Continuation of item 2 of first sheet)**

This international search report has not been established in respect of certain claims under Article 17(2)(a) for the following reasons:

1. [X] Claims Nos.: 35-46
because they relate to subject matter not required to be searched by this Authority, namely:
The inventions as set forth in these claims are relevant to methods for treatment of the human body by therapy.
(Article 17(2)(a)(i) of the PCT, Rule 39.1(iv) of the Regulations under the PCT)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9636624 A
- WO 9916768 A
- WO 9501338 A
- WO 0014085 A
- WO 9414742 A
- WO 9955696 A
- WO 9219594 A
- US 3636039 A
- WO 8706576 A
- WO 9822455 A
- WO 9517386 A
- WO 8705676 A
- EP 389282 A

### Non-patent literature cited in the description

- *Br. J. Pharmacol.,* 1997, vol. 121, 221
- *J. Invest. Dermatol.,* vol. 84, 477
- *J. Pharmacol. Exp. Ther.,* 1994, vol. 271, 1167
- *J. Invest. Dermatol.,* 1998, vol. 110, 287
- *Mol. Pharmacol.,* 1995, vol. 47, 1164
- *Clin. Exp. Allergy,* 1995, vol. 25, 616
- *J. Allergy Clin. Immunol.,* 2001, vol. 107, 871
- *Folia Pharmacol. Jpn.,* 2001, vol. 117, 351-357
- *Lancet,* 2003, vol. 361, 151-160
- *Expert Opin. Emerging Drugs,* 2002, vol. 7, 69-90
- *Eur. J. Pharmacol.,* 2002, vol. 446, 195
- *J. Pharmacol. Exp. Ther.,* 1998, vol. 287, 705
- *J. Med.Chem.,* 1994, vol. 37, 1696
- *J. Med.Chem.,* 1998, vol. 41, 821
- *J. Med.Chem.,* 1998, vol. 41, 2268
- *Br. J. Dermatol.,* 2002, vol. 147, 299
- *J. Am. Acad. Dermatol.,* 1999, vol. 41, 72
- *Exp. Opin. Invest. Drugs,* 1999, vol. 8, 1301-1325
- *J. Med. Chem.,* 1994, vol. 37, 1696
- *J. Med. Chem.,* 1998, vol. 41, 821
- *J. Med. Chem.,* 1998, vol. 41, 2268
- *Cancer,* 1997, vol. 80, 1141